# EUROPÄISCHE PATENTSCHRIFT

(11) **EP 3 270 795 B1**
(45) Veröffentlichungstag und Bekanntmachung des Hinweises auf die Patenterteilung: **15.05.2019**
(21) Anmeldenummer: 16711800.9
(22) Anmeldetag: 15.03.2016
(51) Int. Cl.: A61B 17/04

(54) **ANKERANORDNUNG UND CHIRURGISCHES INSTRUMENT ZUM SETZEN EINER ANKERANORDNUNG**
ANCHOR ARRANGEMENT AND SURGICAL INSTRUMENT FOR SETTING AN ANCHOR ARRANGEMENT
DISPOSITIF D'ANCRAGE ET INSTRUMENT CHIRURGICAL POUR PLACER UN DISPOSITIF D'ANCRAGE

(30) Priorität: 17.03.2015 DE 202015002244 U
(43) Veröffentlichungstag der Anmeldung: 24.01.2018
(73) Patentinhaber: H & B Electronic GmbH & Co. KG, 75392 Deckenpfronn (DE)
(72) Erfinder: WEBER, Wilfried, 72296 Schopfloch (DE); STAUSS, Wolfgang, 72414 Rangendingen (DE); MORLOK, Tobias, 71159 Mötzingen (DE)
(74) Vertreter: Wacker, Jost Oliver
(86) Internationale Anmeldenummer: PCT/EP2016/055513
(87) Internationale Veröffentlichungsnummer: WO 2016/146615

(56) Entgegenhaltungen:
- US-A1- 2006 190 042
- US-A1- 2007 276 412
- US-A1- 2011 071 549

## Beschreibung

Die Erfindung betrifft eine Ankeranordnung zur chirurgischen Gewebereparatur, wie insbesondere zur Reparatur eines Meniskusrisses, nach dem Anspruch 1. Ferner betrifft die Erfindung ein chirurgisches Instrument zum Setzen einer Ankeranordnung. Die Ankeranordnung weist dabei wenigstens einen ersten Anker und einen zweiten Anker auf, die für die jeweilige Platzierung an dem zu reparierenden Gewebe entlang einer Hohlnadel verschoben und aus dieser heraus ausgeworfen werden können. Die wenigstens zwei Anker sind dabei über ein Nahtelement miteinander verbunden. Zur Reparatur des betreffenden Gewebes kann dabei zumindest ein die beiden Anker verbindender Teil des Nahtelementes verkürzt werden, wodurch beispielsweise ein Riss des Gewebes geschlossen werden kann. Der erste Anker und der zweite Anker erstrecken sich in jedem Fall zwischen einem distalen Ende und einem proximalen Ende und bilden an ihrer Außenseite eine Führungsfläche, die zur Führung entlang einer Innenseite der Hohlnadel dient. Dabei sind an den Ankern Umlenkmittel vorgesehen, über die die Anker beim oder nach dem Setzen wenigstens teilweise mit einem Drehmoment beaufschlagt werden können. Durch die vorgesehenen Umlenkmittel ist es dabei möglich, die Anker noch innerhalb der Hohlnadel oder beim beziehungsweise nach dem Austritt aus der Nadelspitze mit einem Drehmoment zu beaufschlagen, um den Anker insgesamt oder Teile desselben in eine von der Setzrichtung abweichende Richtung zu verlagern. Hierdurch können die Anker zumindest teilweise querstehend zur Hohlnadel beziehungsweise zu einer mittels der Hohlnadel erzeugten Durchtrittsöffnung im Gewebe verlagert werden. Durch diese wenigstens bereichsweise querstehende Ausrichtung kann somit verhindert werden, dass die Anker bei Zugaufbringung an dem Nahtelement wieder durch die Durchtrittsöffnung zurückgezogen werden.

Aus US 2013/0345751 A1 sind eine Ankeranordnung und eine Setzvorrichtung zur Befestigung von Gewebe an einem Knochen, ohne die Notwendigkeit der vorherigen Einbringung eines Bohrlochs in den Knochen bekannt. Hierzu weist die Ankeranordnung mehrere hintereinander aus der Setzvorrichtung auswerfbare Anker mit einer zylindrischen Außenfläche auf. In diese ist jeweils eine gegenüber einer Längsachse versetzt angeordnete Bohrung zur Befestigung eines schnurförmigen Nahtelementes sowie ein durch eine Materialausnehmung gebildeter Verkantungsabschnitt eingelassen. Nach dem Setzen können die Anker somit durch Zug am Nahtelement mit einem Drehmoment beaufschlagt werden, mittels dem sie sich in einem durch die Setzvorrichtung erzeugten Bohrloch verkanten können.

Aus EP 1 408 848 B1 ist eine Ankeranordnung bekannt, die mittels einer Hohlnadel eines chirurgischen Instrumentes gesetzt werden kann. Um die Anker in einer vorbestimmten Ausrichtung setzen zu können, weist die Hohlnadel hierbei einen in die Nadelspitze einmündenden Längsschlitz auf. Entlang dieses Schlitzes ist dabei eine Finne geführt, die jeweils von einem Grundkörper der Anker absteht. Zudem sind in die Finnen Ausnehmungen eingelassen, an denen ein die Anker verbindendes Nahtelement angebracht ist. US 2007/276412 beschreibt eine Vorrichtung nach der Präambel des Anspruch 1. US 2011/071549 beschreibt ein Instrument zum Setzen einer Ankervorrichtung.

Nachteilig an der bekannten Ankeranordnung ist, dass beim Durchdringen des zu reparierenden Gewebes, dieses teilweise durch die von der Hohlnadel abstehenden Finnen verdrängt werden muss. Zudem können beim Durchdringen des Gewebes Gewebepartikel an der Nadelspitze und am Schlitz aufgenommen und vom übrigen Gewebe abgetrennt werden, die dadurch nicht mehr für die spätere Ausheilung des Gewebematerials zur Verfügung stehen. Darüber hinaus kommt es beim Setzen derartiger Ankeranordnungen mittels einer Hohlnadel häufig vor, dass die Anker nach dem Durchdringen des Gewebes und dem Setzen aus der Hohlnadel heraus bei Zugaufbringung an dem Nahtelement wieder durch die Durchdringungsstelle aus dem Gewebe heraus gezogen werden.

Die Aufgabe der Erfindung ist es, bei einer gattungsgemäßen Ankeranordnung die genannten Nachteile zu vermeiden und ein exaktes Setzen sowie eine minimierte Beanspruchung des betreffenden Gewebes zu gewährleisten.

Diese Aufgabe wird durch eine Ankeranordnung mit den Merkmalen des Anspruchs 1 gelöst. Dabei weist wenigstens einer der Anker wenigstens zwei relativ zueinander umlenkbare beziehungsweise verschwenkbare Ankerabschnitte auf, wobei ein in einer Einklappstellung der Ankerabschnitte an einer Innenseite der Hohlnadel anlegbarer Teil der Führungsfläche einen von einem wenigstens abschnittsweisen Kreisprofil abweichenden Querschnitt aufspannt beziehungsweise ausbildet. Die Teile des Ankers, die in der Einklappstellung dessen Führungsfläche bilden, spannen dadurch senkrecht zur Setzrichtung eine virtuelle Kontur auf, die von einer zylindrischen Form abweicht. Hierdurch kann der jeweilige Anker in dessen Einklappstellung bezüglich einer Längsachse der Hohlnadel exakt in einer bestimmten Drehstellung gegenüber der entsprechend geformten Hohlnadel geführt werden, ohne dass es hierfür weiterer Führungselemente bedarf. Auf diese Weise können die Anker eine relativ kompakte Form aufweisen und die Hohlnadel kann relativ dünn ausgeführt sein, wodurch wiederum eine minimierte Beanspruchung des zu reparierenden Gewebes gewährleistet werden kann. Durch die relativ zueinander verschwenkbaren Ankerabschnitte kann der Anker zudem zwischen der besonders kompakten Form in der Einklappstellung, in der er entlang einer relativ dünnen Hohlnadel verlagert werden kann, und einer aufgeweiteten Form in der Aufklappstellung versteilt werden, in der die verschwenkbaren Ankerabschnitte einen Wiedereintritt in eine Durchtrittsöffnung des betreffenden Gewebes verhindern. Durch diese Klappfunktion wird somit gewährleistet, dass der Anker nach einem Setzvorgang bei Zugaufbringung an dem Nahteiement nicht mehr durch die zuvor hergestellte Durchstechöffnung des Gewebes zurückgezogen werden kann. Dabei sind die wenigstens zwei Ankerabschnitte über ein Gelenk miteinander verbunden. Das Gelenk ermöglicht dabei ein besonders leichtes Umlenken beziehungsweise Aufklappen der betreffenden Ankerabschnitte. Dadurch kann der Anker bereits durch eine sehr geringe Drehmomentbeaufschlagung in die Aufklappstellung verbracht werden. Alternativ oder zusätzlich hierzu sind wenigstens zwei Ankerabschnitte einteilig ausgebildet und über ein Filmscharnier miteinander verbunden. Hierdurch können die betreffenden Ankerabschnitte des Ankers gemeinsam einteilig hergestellt werden. Dies ermöglicht einerseits geringere Herstellungskosten und andererseits kann der Anker hierdurch insgesamt relativ stabil ausgeführt werden.

In einer besonders vorteilhaften Ausführungsform bildet die Führungsfläche in der Einklappstellung der zueinander verlagerbaren Ankerabschnitte ein herzförmiges, ovales oder eiförmiges, im Sinne von umlaufend konvexes beziehungsweise streng konvexes, Profil aus. Durch ein derartiges Profil der Führungsfläche beziehungsweise durch eine derartige seitens der Führungsfläche aufgespannte Außenkontur des Ankers in der Einklappstellung weist dieser trotz seiner unverdrehbaren Führung entlang einer entsprechend geformten Nadel eine sehr kompakte Form auf, die ein besonders exaktes und gleichzeitig gewebeschonendes Setzen ermöglicht.

Zudem ist es besonders günstig, wenn die wenigstens zwei Ankerabschnitte in Richtung der Aufklappstellung vorgespannt sind, wodurch der Anker beim Setzen beziehungsweise unmittelbar nach dem Austritt aus der Hohlnadel selbsttätig in die Aufklappstellung verbracht werden kann. Zusätzlich hierzu können die Ankerabschnitte eine Aufnahmeöffnung aufweisen, durch die das Nahtelement jeweils hindurchgeführt wird. Auf diese Weise kann das Nahtelement zusätzlich zum Verschwenken der Ankerabschnitte genutzt werden. Zusätzlich kann das Nahtelement hierdurch zur Sicherung gegen das Verlieren einzelner Ankerabschnitte dienen. Dabei ist es günstig, wenn das Nahtelement an wenigstens zwei Ankerabschnitten derart umgelenkt ist, dass diese über das Nahtelement mit einem Aufklappmoment beaufschlagt werden können. Auf diese Weise kann die Person, die die Ankeranordnung setzt, durch Aufbringung einer Zugkraft am Nahtelement selbst bestimmen, zu welchem Zeitpunkt die Anker in die vollständige Aufklappstellung verbracht werden.

In einer weiteren vorteilhaften Ausführungsform sind die wenigstens zwei Ankerabschnitte in der Aufklappstellung jeweils durch einen Endanschlag des jeweils anderen Ankerabschnittes abgestützt, wodurch eine besonders stabile Endstellung in der Aufklappstellung ermöglicht wird. Zudem ist es günstig, wenn die wenigstens zwei Ankerabschnitte in der Aufklappstellung verriegelbar beziehungsweise verrastbar sind, wodurch eine ungewollte teilweise oder vollständige Rückstellung beziehungsweise Rückverformung des Ankers in die Einklappstellung, wie beispielsweise beim Platzieren des Ankers an dem zu reparierenden Gewebe verhindert werden kann. Dabei ist es günstig, wenn wenigstens einer der Anker eine raue Oberfläche zur Anlage an dem zu reparierenden Gewebematerial aufweist. Durch diese raue Oberfläche kann nach dem Setzen eine zusätzliche Positionsstabilität des an dem Gewebe anliegenden Ankers gewährleistet werden. Zudem ist es von Vorteil, wenn die wenigstens zwei Ankerabschnitte in der Aufklappstellung eine konkave Kontaktfläche zur Anlage an das Gewebe bilden. Hierdurch kann der Anker auch an einer konvexen Oberfläche des zu reparierenden Gewebes, wie sie beispielsweise bei der Reparatur eines Meniskus vorliegen kann, wenigstens über einen Großteil seiner Länge hinweg in Anlage gebracht werden. Ferner ist es günstig, wenn die wenigstens zwei Ankerabschnitte an einem jeweiligen freien Ende jeweils eine Abrundung aufweisen. Durch die Abrundungen kann vermieden werden, dass sich die freien Enden der Ankerabschnitte vor Erreichen der Aufklappstellung an dem betreffenden Gewebe festsetzen.

In einer weiteren vorteilhaften Ausführungsform bilden die wenigstens zwei Ankerabschnitte in der Einklappstellung am proximalen Ende des Ankers eine gemeinsame Spreizaufnahme aus, die durch Beaufschlagung mittels eines Spreizelementes aufspreizbar ist. Hierdurch können die beiden Ankerabschnitte durch eine aktive Beaufschlagung der Spreizaufnahme insbesondere während des Setzvorganges relativ zueinander umgelenkt und dadurch rechtzeitig vor Beaufschlagung des Ankers mit einer Zugkraft über das Nahtelement sicher in die Aufklappstellung verbracht werden. Zudem ist es günstig, wenn die Anker und/oder das Nahtelement wenigstens teilweise durch ein mittels eines Impulses aktivierbares resorbierbares Material gebildet sind. Auf diese Weise können wenigstens Teile der Ankeranordnung nach einer erfolgreichen Reparatur mittels eines Impulses aktiviert werden, um anschließend im Körper abgebaut zu werden. Der Impuls kann dabei je nach Art des verwendeten Materials beispielsweise aus einem Magnet-, Temperatur- oder Lichtimpuls bestehen. In jedem Fall ist hierdurch eine weitestgehend vollständige Verheilung des betreffenden Körperbereichs ohne Rückstände der Ankeranordnung möglich. In einer weiteren vorteilhaften Ausführungsform ist wenigstens einer der Anker wenigstens teilweise durch ein Formgedächtnismaterial gebildet, das durch Licht, Temperatur oder ein elektrisches oder magnetisches Feld aktiviert werden kann. Hierdurch können die betreffenden Ankerabschnitte auch ohne Kraftaufbringung in die Aufklappstellung des Ankers umgelenkt werden. Zudem kann auch hier der Zeitpunkt, zu dem der Anker die Aufklappstellung einnimmt, von der die Ankeranordnung setzenden Person frei bestimmt werden. Hierbei ist es besonders vorteilhaft, wenn das Formgedächtnismaterial wenigstens zwei Verformungsabschnitte aufweist, die zeitlich nacheinander in eine jeweilige Endstellung verformbar sind. Auf diese Weise lässt sich eine bestimmte Abfolge vorgeben, in der verschiedene Abschnitte des Ankers umgelenkt werden.

Ferner wird die oben genannte Aufgabe durch ein chirurgisches Instrument zur chirurgischen Gewebereparatur, wie insbesondere zur Reparatur eines Meniskusrisses gelöst, das zum Setzen einer Ankeranordnung in einer der oben genannten Ausführungsformen dient. Das chirurgische Instrument weist dabei eine Hohlnadel, die an einem distalen Ende eine Nadelspitze ausbildet, und eine Auswurfeinrichtung mit einem innerhalb der Hohlnadel verlagerbaren Auswurfelement auf. Mittels dieses Auswurfelementes können der erste und zweite Anker entlang der Hohlnadel verschoben werden, wobei die Hohlnadel an der Nadelspitze einen geschlossen umlaufenden oder schließbaren Querschnitt aufweist, das heißt dass der Querschnitt der Hohlnadel an der Nadelspitze entweder stofflich geschlossen oder über wenigstens einen Stoß umlaufend geschlossen ausgebildet ist. Zudem weist die Hohlnadel wenigstens zur Nadelspitze hin einen von einem Kreisprofil abweichenden, vorzugsweise umlaufend konvexen Querschnitt auf, wie insbesondere einen elliptischen, ovalen, ei- oder herzförmigen Querschnitt oder einen insbesondere an eine dieser Formgebungen angenäherten polygonförmigen Querschnitt. Durch die umlaufend geschlossene Nadelspitze kann die Beanspruchung des zu reparierenden Gewebes beim Setzen der Anker auf ein Minimum reduziert werden. Insbesondere kann durch die geschlossene Ausbildung der Nadelspitze die Abtrennung von Gewebepartikeln beim Durchdringen des Gewebes weitestgehend vermieden werden, was wiederum eine schnellere Gewebeverheilung ermöglicht. Zudem ermöglicht die geschlossene Ausbildung der Nadelspitze eine erhöhte Stabilität der Hohlnadel insgesamt. Durch den von einem Kreisprofil abweichenden Querschnitt kann das Gewebe, das von der Hohlnadel zum Setzen der Anker durchdrungen wird, einen gewissen Widerstand gegen ein Verdrehen der Hohlnadel um deren Längsachse herum ausüben, so dass die Hohlnadel beim Durchdringen des Gewebes in gewisser Weise durch das Gewebe selbst geführt ist. Zudem gewährleistet der von einem Kreisprofil abweichende Querschnitt der Hohlnadel und die daran angepassten Führungsflächen der Anker eine exakte Führung derselben in einer vorgegebenen Drehstellung. Aufgrund dieser vorgegebenen Drehstellung der Anker gegenüber der Hohlnadel kann der Anwender dabei beim Setzvorgang die Anker derart auswerfen, dass sie entlang einer gewünschten Richtung aufklappen. Darüber hinaus kann durch diese Formgebung zumindest hinsichtlich einer Bezugsebene eine höhere Biegesteifigkeit der Hohlnadel erreicht werden. Hierbei kann die Hohlnadel beispielsweise auch stofflich geschlossen ausgebildet sein und eine Längsnut zur wenigstens teilweisen Aufnahme des Nahtelementes aufweisen. Durch diese Aufnahme des Nahtelementes in einer hierfür vorgesehenen Längsnut ist eine störungsfreie Führung der Anker innerhalb der Hohlnadel möglich. Zudem kann durch die geschlossene Ausbildung über die gesamte Länge der Hohlnadel hierbei eine besonders hohe Biegesteifigkeit derselben gewährleistet werden.

In einer vorteilhaften Ausführungsform weist die Hohlnadel alternativ oder zusätzlich zu der Längsnut einen Längsschlitz auf, wobei zwei sich gegenüberliegende Ränder des Längsschlitzes im unbelasteten Zustand wenigstens an der Nadelspitze aneinander liegen. Hierdurch kann eine geschlossene Hohlnadel zur Verfügung gestellt werden, deren Längsschlitz lediglich zeitweise geöffnet wird, um beispielsweise den Faden zusammen mit den Ankern setzen zu können. Durch die aneinander liegenden Ränder kann dabei trotz des Schlitzes eine relativ hohe Stabilität der Hohlnadel beibehalten und ein ungewolltes Eindringen und Abtrennen von Gewebematerial während eines Einstiches vermieden werden.

Hierbei ist es günstig, wenn die Ränder mittels Einpressen eines Abschnittes des Nahtelementes oder eines Abschnittes der Anker aufweitbar sind, wodurch der Längsschlitz lediglich während der Verlagerung der Anker entlang der Hohlnadel geöffnet wird und die Hohlnadel im Übrigen geschlossen ist. Dabei ist es zudem günstig, wenn die Hohlnadel zur Nadelspitze hin eine Krümmung aufweist, wodurch bei bestimmten Anwendungen des chirurgischen Instrumentes ein zum Setzen der Anker vorgesehener Bereich des betreffenden Gewebes leichter zugänglich gemacht werden kann.

In einer besonders vorteilhaften Ausführungsform des chirurgischen Instrumentes kann die Nadelspitze durch den ersten und den zweiten Anker einer darin aufgenommenen Ankeranordnung nach einer der oben genannten Ausführungsformen jeweils bündig verschlossen werden. Auf diese Weise können die wenigstens zwei zum Setzen vorgesehenen Anker die Hohlnadel des chirurgischen Instrumentes ähnlich einem Pfropfen verschließen und dadurch verhindern, dass beim Durchdringen des Gewebes Gewebepartikel an der Nadelspitze in die Hohlnadel gelangen und vom übrigen Gewebe abgetrennt werden. Hierzu weisen die Anker am distalen Ende eine Schräge auf, die gegenüber einer Längsachse des Grundkörpers einen mit der distalen Nadelspitze übereinstimmenden Anstellwinkel aufweist. Hierdurch können die Anker zusammen mit der Nadelspitze ein geschlossenes distales Ende der Hohlnadel bilden, in das während eines Einstichs und Durchdringens eines Gewebes keine Gewebepartikel in die Hohlnadel eindringen können. Hierbei ist vorgegeben, ob die wenigstens zwei Anker bezogen auf eine für eine Anwendung des chirurgischen Instruments vorgesehene Winkelstellung bezüglich einer Setzachse in vertikaler Richtung oder alternativ hierzu in horizontaler Richtung aufklappbar sind. Hierdurch kann je nach Anwendungsfall eine bevorzugte Aufklapprichtung ausgewählt werden, mittels der eine optimierte Festlegung der Anker an dem betreffenden Gewebe erzielt werden kann. Ferner ist es günstig, wenn das Auswurfelement an einem distalen Ende Aufspreizmittel zur Beaufschlagung des proximalen Endes des ersten Ankers und/oder des zweiten Ankers aufweisen. Hierdurch ist es möglich, insbesondere Anker mit wenigstens zwei zueinander verlagerbaren Ankerabschnitten am proximalen Ende mittels des Auswurfelementes mit Spreizkräften zu beaufschlagen. Auf diese Weise können die wenigstens zwei Ankerabschnitte beim Setzvorgang durch das Auswurfelement aktiv aufgespreizt beziehungsweise aufgeklappt werden, um einen Wiedereintritt in die Hohlnadel beziehungsweise in die durch die Hohlnadel erzeugte Durchtrittsöffnung im Gewebe vermeiden zu können. Vorteilhafterweise weisen die Aufspreizmittel einen Verjüngungsbereich am distalen Ende des Auswurfelementes auf, der zwischen die zwei Ankerabschnitte des ersten Ankers und/oder des zweiten Ankers verlagerbar ist. Auf diese Weise können die beiden zueinander verlagerbaren Ankerabschnitte eines Ankers durch bloßes Einpressen des Verjüngungsbereiches am proximalen Ende des betreffenden Ankers voneinander weg verlagert beziehungsweise aufgespreizt werden. Alternativ oder zusätzlich hierzu können die Aufspreizmittel wenigstens zwei am distalen Ende des Auswurfelementes angeordnete elastische Spreizarme aufweisen, die in Anlage an die Ankerabschnitte eines der Anker voneinander weg vorspannbar sind. Durch derartige vorgespannte Spreizarme kann beim Setzen der Anker ein sicheres Aufspreizen beziehungsweise Aufklappen der beiden zueinander verlagerbaren Ankerabschnitte gewährleistet werden. In einer hierzu alternativen Ausführungsform weist die Auswurfeinrichtung für den ersten Anker und den zweiten Anker jeweils einen separaten Auswerfer auf, wodurch beispielsweise für jeden Anker ein eigener Auswurfmechanismus beziehungsweise ein eigenes Betätigungselement vorgesehen werden kann.

In einer weiteren vorteilhaften Ausführungsform des chirurgischen Instrumentes sind an der Hohlnadel Spannmittel zum Spannen des Nahtelementes vorgesehen. Durch derartige Spannmittel kann vermieden werden, dass es insbesondere beim Durchdringen des zu reparierenden Gewebes aufgrund des an der Hohlnadel mitgeführten Nahtelementes zu Störungen oder zu einem zusätzlichen Abtrennen von Gewebepartikeln kommt. Dabei ist es günstig, wenn die Spannmittel ein entlang der Hohlnadel verschiebbares Schiebeelement aufweisen, an dem das Nahtelement umgelenkt ist und das in proximaler Richtung vorgespannt ist. Hierdurch kann das Nahtelement besonders platzsparend an der Hohlnadel gehalten und in einfacher Weise beim Setzen der Anker freigegeben werden.

Vorteilhafterweise weist die Hohlnadel an der Nadelspitze einen distalen Abschnitt und einen hierzu proximal angeordneten Aufnahmeabschnitt auf, der gegenüber dem distalen Abschnitt einen erweiterten Querschnitt ausbildet. Hierdurch ist es möglich, den distalen Abschnitt möglichst dünn auszuführen, um darin lediglich die Anker zur Nadelspitze hin verlagern zu können, und im proximalen Aufnahmeabschnitt neben dem oder den hierin aufgenommenen Ankern auch Teile der Auswurfeinrichtung unterzubringen.

Dabei ist es besonders günstig, wenn in dem erweiterten Querschnitt das Auswurfelement an dem zweiten Anker vorbei geführt werden kann. Auf diese Weise kann beispielsweise der Erste Anker mittels des Auswurfelementes entlang des distalen Abschnittes zur Nadelspitze verlagert und dort ausgeworfen werden, während der zweite Anker und gegebenenfalls weitere Anker in einer passiven Stellung im proximalen Aufnahmeabschnitt verbleiben können. Alternativ hierzu ist es auch möglich, dass der proximale Abschnitt mit einer Griffaufnahme eines Instrumentengriffes verbunden ist, in dem wenigstens ein Anker vorab aufgenommen werden kann. Auf diese Weise ist es möglich, an dem chirurgischen Instrument eine Vielzahl von Ankern zwischen zu speichern und diese gegebenenfalls gegen äußere Beeinflussungen abzuschirmen. Hierdurch können beispielsweise auch Anker an dem chirurgischen Instrument mitgeführt werden, die ein durch Temperatur, Licht oder Elektrizität aktivierbares Formgedächtnismaterial aufweisen. Dabei ist es günstig, wenn der wenigstens eine Anker, durch einen vorspannbaren Anker gebildet ist und im proximalen Aufnahmeabschnitt der Hohlnadel oder in der Griffaufnahme in einer spannungsfreien Stellung aufgenommen werden kann. Hierdurch ist es möglich, den Anker auch über eine relativ lange Zeit vor dem Setzen in dem chirurgischen Instrument mitzuführen, ohne dass sich die elastischen Mittel, mittels denen die Vorspannung hergestellt werden kann und die beispielsweise durch ein Federelement oder einen elastischen Materialabschnitt gebildet sind, relaxieren können. Hierzu werden die Anker in einer wenigstens annähernd spannungsfreien Stellung in der Griffaufnahme gelagert und jeweils erst kurz vor ihrem Setzvorgang in ihre vorgespannte Stellung verbracht, in der sie entlang der Hohlnadel zur Nadelspitze hin verschoben werden können. Zudem ist es dabei günstig, wenn der wenigstens eine Anker von der Griffaufnahme in den proximalen Abschnitt verschoben werden kann, wodurch der Anker in einer besonders komfortablen Weise von der vorgelagerten Stellung in der Griffaufnahme in eine aktivierbare Stellung innerhalb der Hohlnadel übergeführt werden kann, aus der heraus er mittels der Auswurfeinrichtung verlagert werden kann, ohne dass der Anker hierbei beispielsweise händisch aus der Griffaufnahme entnommen werden muss.

Zudem ist es vorteilhaft, wenn in dem proximalen Aufnahmeabschnitt Festlegemittel vorgesehen sind, mittels denen der zweite Anker in beide axiale Richtungen abgestützt werden kann. Hierdurch kann der zweite Anker während eines Setzvorganges des ersten Ankers sicher in der passiven Stellung gehalten werden, um Störungen beim Setzen des ersten Ankers zu vermeiden.

In einer besonders vorteilhaften Ausführungsform des chirurgischen Instrumentes weist dieses ein Zahnradgetriebe zur Steuerung der Bewegungsabläufe der Auswurfeinrichtung auf. Auf diese Weise können die einzelnen Bewegungsabläufe zum aufeinanderfolgenden Setzen der wenigstens zwei Anker, wie beispielsweise das Verlagern des zweiten Ankers in eine setzbereite Position nach dem Setzen des ersten Ankers oder die distalen und proximalen Verlagerungen des wenigstens einen Auswurfelementes, in ihrer Abfolge oder ihrem zeitlichen Verlauf besonders exakt gesteuert werden. Dabei kann die Auswurfeinrichtung mittels einer benachbart zu einem Instrumentengriff angeordneten Betätigungselement betätigt werden, die von einer Ausgangsstellung in eine Setzstellung manuell verlagert werden kann. Auf diese Weise kann die Auswurfeinrichtung während des gesamten Setzvorganges vom Operateur manuell gesteuert werden. Dabei ist es vorteilhaft, wenn das Zahnradgetriebe eine vorbestimmte Übersetzung zwischen einem manuell oder mittels eines Kraftspeichers verlagerbaren Betätigungselement und dem Auswurfelement autweist. Je nachdem welche Bewegungsgeschwindigkeit aus der zu erwartenden Kraftbeaufschlagung des Betätigungselementes resultiert, kann auf diese Weise eine besonders geeignete Verlagerungsgeschwindigkeit des Auswurfelementes vorbestimmt werden. Alternativ hierzu ist es auch möglich, dass die Übersetzung zwischen dem Betätigungselement und dem Auswurfelement eingestellt werden kann, wobei insbesondere eine von mehreren einstellbaren Übersetzungen ausgewählt werden kann. Hierdurch kann eine das chirurgische Instrument verwendende Person beziehungsweise ein Operateur das Instrument auf seine persönlichen Ansprüche beziehungsweise an eine vorgesehene Anwendung anpassen.

Vorteilhafterweise können die Anker jeweils in einer setzbereiten Position in proximaler Richtung durch die Auswurfeinrichtung abgestützt werden, wobei die Bewegung der Auswurfeinrichtung in proximaler Richtung gesperrt ist. Auf diese Weise kann vermieden werden, dass die Anker in der setzbereiten Position insbesondere beim Einstechen der Hohlnadel in das zu reparierende Gewebe von diesem in proximaler Richtung verschoben werden, wodurch wiederum Gewebematerial an der Nadelspitze in die Hohlnadel eindringen könnte.

Zudem ist es günstig, wenn das Betätigungselement an einen Endanschlag anlegbar ist und die Auswurfeinrichtung dabei in einer Endanschlagstellung angeordnet ist, die der setzbereiten Position des jeweils mitgeführten Ankers entspricht. Hierdurch kann dem Anwender bei der manuellen Betätigung des chirurgischen Instrumentes der setzbereite Zustand des jeweiligen Ankers sicher angezeigt und ein versehentliches Setzen desselben verhindert werden.

Ferner ist es von Vorteil, wenn das Betätigungselement unter Trennung des Zahnradgetriebes von der Endanschlagstellung in eine Freigabestellung verdreht werden kann, in der das Betätigungselement weiter in Setzrichtung verlagerbar ist. Hierdurch ist es möglich, nach dem Erreichen der setzbereiten Position des zu setzenden Ankers, die zwischen Betätigungselement und Instrumentengriff wirkende Übersetzung aufzuheben. Hierdurch kann beispielsweise die Verlagerung eines Ankers vom proximalen Aufnahmeabschnitt in die setzbereite Position mit einer relativ großen Übersetzung und dadurch durch eine lediglich geringe Verlagerung des Betätigungselementes erfolgen. Nach Erreichen der Endanschlagstellung und Verdrehen des Betätigungselementes in die Freigabestellung erfolgt dann eine direkte Verlagerung der Auswurfeinrichtung über das Betätigungselement ohne Übersetzung, um dem Anwender eine bessere Steuerung des Setzvorganges zu ermöglichen.

Vorteilhafterweise sind die Endanschlagstellung und die Freigabestellung dabei durch eine am Instrumentengriff vorgesehene Steuerkurve vorgegeben, in die ein mit dem Betätigungselement mitgeführter Nocken hinein ragt. Hierdurch können die Endanschlagstellung und die Freigabestellung in einfacher und exakter Weise festgelegt werden.

Ferner ist es günstig, wenn ein Auswurfendanschlag vorgesehen ist, mittels dem die Bewegung der Auswurfeinrichtung in distaler Richtung begrenzt werden kann, um zu vermeiden, dass das Auswurfelement aus der Hohlnadel heraus austreten kann beziehungsweise dass eine Fehlfunktion in Folge einer übermäßigen Beanspruchung der Auswurfeinrichtung auftritt. Vorteilhafterweise ist das Betätigungselement nach dessen Betätigung und Freigabe durch eine betätigende Hand mittels einer Rückstellfedereinrichtung von der Auswurfendanschlagstellung in die Ausgangsstellung verlagerbar, wobei gleichzeitig die Auswurfeinrichtung hinter den zweiten Anker verfahren werden kann. Hierdurch muss der Operateur während einer Anwendung lediglich das Setzen der Anker manuell vornehmen, während nach dem Setzen des ersten Ankers der zweite Anker selbsttätig in eine setzbereite Position verbracht wird. Auf diese Weise wird die Anwendung des chirurgischen Instrumentes für den Operateur insgesamt komfortabler. Vorteilhafterweise ist dabei ein Signalgeber vorgesehen, der beim Erreichen der Ausgangsstellung selbsttätig aktiviert werden kann. Auf diese Weise kann dem Operateur während einer Anwendung beispielsweise eine akustische, haptische oder optische Rückmeldung gegeben werden, wenn sich der zweite Ankes in der setzbereiten Position befindet. Auf diese Weise erhält der Operateur eine verlässliche Rückmeldung, sobald das chirurgische Instrument nach Setzen eines Ankers bereit ist, um auch den nachfolgenden Anker setzen zu können.

In den Figuren ist eine beispielhafte Ausführungsform der Erfindung dargestellt. Es zeigen:
- Figur 1: eine perspektivische Ansicht einer erfindungsgemäßen Ankeranordnung,
- Figur 2: eine geschnittene Ansicht einer Ankeranordnung im gesetzten Zustand an einem zu reparierenden Gewebe,
- Figur 3: eine geschnittene Ansicht einer Hohlnadel zum Setzen der Ankeranordnung,
- Figur 4: eine perspektivische Explosionsansicht eines aufklappbaren Ankers der Ankeranordnung nach Fig. 1,
- Figur 5: eine perspektivische Ansicht des Ankers nach Figur 4 in einer teilweise aufgeklappten Stellung,
- Figur 6: eine Ansicht einer alternativen Ausführungsform des aufklappbaren Ankers der Ankeranordnung in einer Einklappstellung,
- Figur 7: eine Ansicht des aufklappbaren Ankers nach Fig. 6 in einer teilweise aufgeklappten Stellung,
- Figur 8: eine Ansicht des aufklappbaren Ankers nach Fig. 6 in einer Aufklappstellung und einem im Gewebe platzierten Zustand,
- Figur 9: eine Ansicht einer weiteren alternativen Ausführungsform des aufklappbaren Ankers mit einer konkaven Anlegefläche,
- Figur 10: eine Ansicht einer weiteren alternativen Ausführungsform des aufklappbaren Ankers mit durchgeschleiftem Nahtelement in Einklappstellung,
- Figur 11: eine perspektivische Ansicht des aufklappbaren Ankers nach Fig. 10 in Aufklappstellung,
- Figur 12: eine perspektivische Ansicht einer weiteren Ausführungsform des aufklappbaren Ankers mit einem Endanschlag,
- Figur 13: eine Ansicht des aufklappbaren Ankers nach Fig. 12 kurz vor Erreichen der Aufklappstellung,
- Figur 14: eine Ansicht einer einteiligen Ausführungsform des klappbaren Ankers mit einem Filmscharnier,
- Figur 15: eine Ansicht des klappbaren Ankers nach Figur 14 in der Aufklappstellung und einem im Gewebe platzierten Zustand,
- Figur 16: eine Ansicht einer weiteren alternativen Ausführungsform des klappbaren Ankers mit einer alternativen Federeinrichtung in der Einklappstellung,
- Figur 17: eine Ansicht des klappbaren Ankers nach Fig. 16 in einem teilweise aufgeklappten Zustand,
- Figur 18: eine Ansicht eines klappbaren Ankers in Einklappstellung mit einem daran angreifenden Aufspreizmittel
- Figur 19: eine Ansicht des klappbaren Ankers nach Fig. 18 in teilweise aufgeklappter Stellung,
- Figur 20: eine Ansicht eines klappbaren Ankers in Einklappstellung mit einem daran angreifenden Aufspreizmittel in einer alternativen Ausführungsform.
- Figur 21: eine Ansicht des klappbaren Ankers nach Fig. 20 in teilweise aufgeklappter Stellung,
- Figur 22: eine perspektivische Ansicht einer Nadelspitze einer alternativen Hohlnadel mit einer Längsnut und einem an der Nadelspitze bündig angeordneten distalen Ende eines Ankers.
- Figur 23: eine Vorderansicht der Nadelspitze nach Fig. 22,
- Figur 24: eine perspektivische Ansicht einer Nadelspitze einer alternativen Ausführungsform der Hohlnadel mit einem Längsschlitz,
- Figur 25: eine perspektivische Ansicht der Hohlnadel nach Fig. 24 mit daran aufgenommener Ankeranordnung,
- Figur 26: eine geschnittene Ansicht der Hohlnadel nach Fig. 25,
- Figur 27: eine perspektivische Ansicht der Hohlnadel eines erfindungsgemäßen chirurgischen Instrumentes mit einem Schiebeelement zum Spannen des Nahtelementes,
- Figur 28: eine Ansicht einer weiteren alternativen Ausführungsform der Hohlnadel mit einem proximalen Aufnahmebereich,
- Figur 29: eine perspektivische Ansicht einer Ankeranordnung und einer alternativen Ausführungsform einer Auswurfeinrichtung,
- Figur 30: eine geschnittene Ansicht eines Instrumentengriffes mit einer Ankeranordnung in Speicherstellung,
- Figur 31: eine geschnittene Ansicht des Instrumentengriffes nach Fig. 37 mit einem in eine passive Stellung verbrachten Anker,
- Figur 32: eine perspektivische Ansicht einer weiteren Ausführungsform eines klappbaren Ankers in der Einklappstellung,
- Figur 33: eine perspektivische Ansicht des klappbaren Ankers nach Fig. 39 in der Aufklappstellung,
- Figur 34: eine perspektivische Ansicht einer manuell zu betätigenden Ausführungsform des chirurgischen Instrumentes in einer Ausgangsstellung,
- Figur 35: eine Ansicht des chirurgischen Instrumentes nach Fig. 34 in einer setzbereiten Stellung,
- Figur 36: eine Ansicht des chirurgischen Instrumentes nach Fig. 34 in einer Setzstellung,
- Figur 37: eine Ansicht des chirurgischen Instrumentes nach Fig. 34 beim Verdrehen in eine Freigabestellung,
- Figur 38: eine perspektivische Ansicht des chirurgischen Instrumentes nach Fig. 34 bei abgenommenem Instrumentengriff,
- Figur 39: eine perspektivische Ansicht des chirurgischen Instrumentes nach Fig. 38 in der setzbereiten Stellung,
- Figur 40: eine perspektivische Ansicht einer Steuerkurve des Instrumentengriffes,
- Figur 41: eine perspektivische Ansicht der Steuerkurve nach Fig. 40 mit daran angeordnetem Betätigungselement,
- Figur 42: eine perspektivische Ansicht des chirurgischen Instrumentes nach Fig. 38 in der Freigabestellung und
- Figur 43: eine perspektivische Ansicht des chirurgischen Instrumentes nach Fig. 38 in einer Auswurfendanschlagstellung.

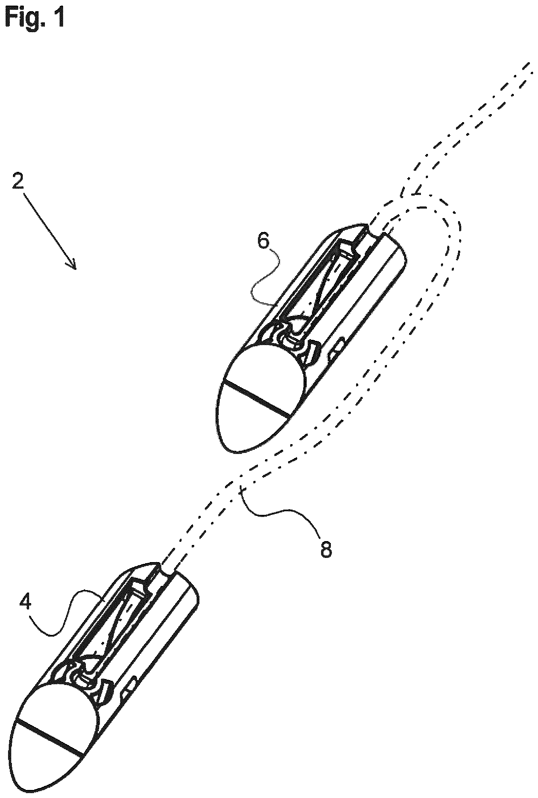

Fig. 1 zeigt eine Ankeranordnung 2 zur Reparatur eines Gewebes G, die hierzu wenigstens einen ersten Anker 4 und einen zweiten Anker 6 aufweist, die mittels eines Nahtelementes 8 miteinander verbunden sind. Das Nahtelement 8 kann dabei beispielsweise aus einem Garn bestehen, das aus einer einzelnen Natur- oder Kunstfaser oder mehreren Natur- oder Kunstfasern hergestellt ist.

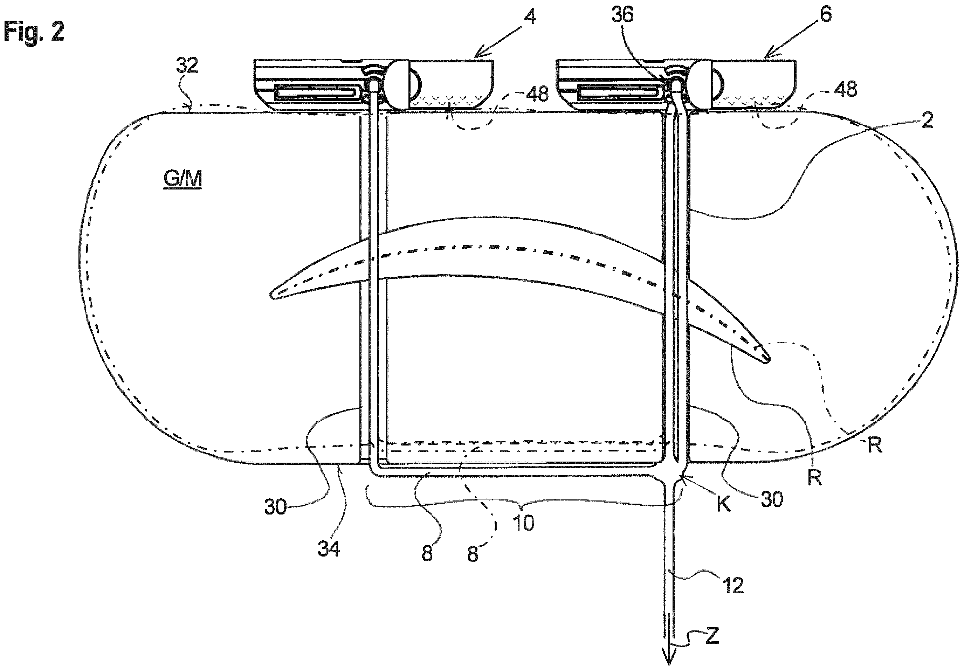

Wie insbesondere aus Fig. 2 zu entnehmen ist, ist das Nahtelement 8 dabei derart an den wenigstens zwei Ankern 4, 6 angebracht, dass ein die beiden Anker 4,6 verbindender Nahtabschnitt 10 nach dem Setzen der Ankeranordnung 2 an dem zu reparierenden Gewebe G, wie beispielsweise einem Meniskus M mit einem Riss R, durch Zugaufbringung Z an einem Ende 12 des Nahtelementes 8 verkürzt werden kann. Hierfür kann in dem Nahtabschnitt 10 beispielsweise ein verschiebbarer Knoten K vorgesehen sein. Durch die Verkürzung des Nahtabschnittes 10 kann dabei der Riss R geschlossen werden, wie durch strichpunktierte Linien dargestellt.

Hierbei können das Nahtelement 8 und/oder die Anker 4, 6 wenigstens teilweise aus einem resorbierbaren Material hergestellt sein. Dabei ist es besonders günstig, wenn das resorbierbare Material mittels eines Impulses aktiviert werden kann, wie beispielsweise durch einen Wärme- oder Lichtimpuls oder durch Anlegen eines magnetischen Feldes. Auf diese Weise kann die Ankeranordnung 2, insbesondere nach einem gewissen Ausheilungsprozess des Risses R, wenigstens teilweise allmählich abgebaut werden.

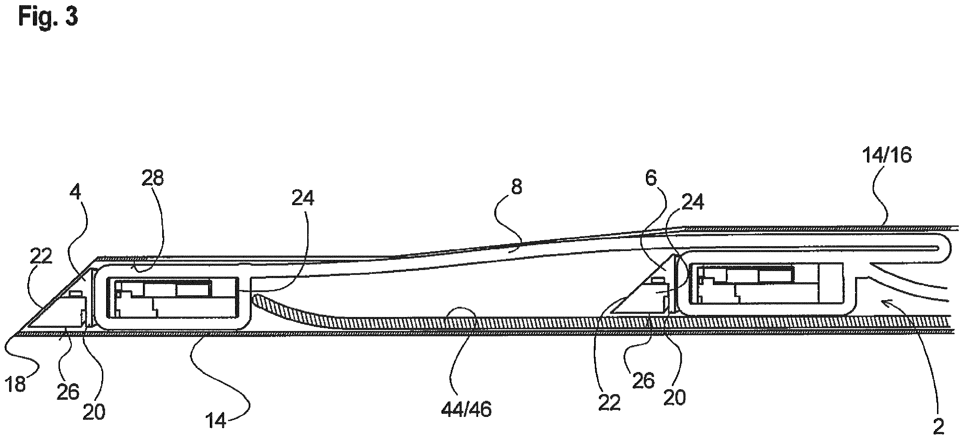

Um die Ankeranordnung 2 an dem zu reparierenden Gewebe G platzieren zu können, sind die wenigstens zwei Anker 4, 6 derart ausgebildet, dass sie zusammen mit dem Nahtelement 8 an beziehungsweise in einer Hohlnadel 14 eines chirurgischen Instruments 16 aufgenommen werden können, wie in Fig. 3 dargestellt. In dieser können sie dann in Richtung einer Nadelspitze 18 verschoben werden, um aus dieser heraus nacheinander an gewünschten Positionen des Gewebes G gesetzt werden zu können.

Hierzu weisen beide Anker 4, 6 jeweils einen Grundkörper 20 auf, der sich von einem jeweiligen distalen Ende 22 zu einem proximalen Ende 24 erstreckt und der dabei an seiner Außenseite eine Führungsfläche 26 bildet, die wenigstens teilweise an eine Innenseite 28 der Hohlnadel 14 anlegbar ist, um beim Verschieben in distaler Richtung zur Nadelspitze 18 hin von dieser lagestabil geführt werden zu können.

Hierdurch können mittels der Hohlnadel 14 Durchtrittsöffnungen 30 durch das Gewebe G gestochen werden, über die die Anker 4, 6 nacheinander an einer ersten Seite 32 des Gewebes G positioniert werden können, wie in Fig. 2 dargestellt. Das Nahtelement 8 erstreckt sich dabei nach dem Setzen von dem ersten Anker 4 weg durch die zugehörige Durchtrittsöffnung 30 hindurch entlang einer zweiten Seite 34 des Gewebes G und durch die Durchtrittsöffnung 30 hindurch zum zweiten Anker 6. An diesem ist dabei eine Ausnehmung 36 vorgesehen durch die hindurch das Nahtelement 8 durchgeschleift wird und sich zurück über die Durchtrittsöffhung 30 zum Ende 12 erstreckt.

Um sicherzustellen, dass die Anker 4, 6 nach dem Austritt aus der Hohlnadel 14 und Aufbringen der Zugkraft Z am Nahtelement 8 nicht wieder in die jeweilige Durchtrittsöffnung 30 eintreten, weisen die Anker 4, 6 Umlenkmittel 38 auf, über die wenigstens Teile der Anker 4, 6 beim Auswerfen, das heißt insbesondere während oder nach dem Austritt aus der Nadelspitze 18 mit einem Drehmoment MD beaufschlagt werden können.

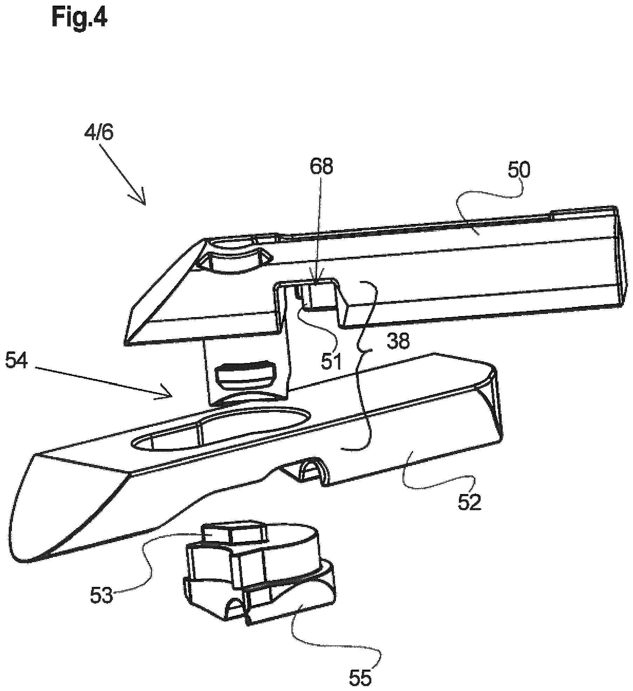

Hierzu sind die wenigstens zwei Anker 4, 6 mehrteilig, insbesondere zweiteilig ausgebildet, wie insbesondere aus Fig. 4 zu entnehmen ist. Die Umlenkmittel 38 sind dabei durch ein Gelenk 54 und eine Federeinrichtung 68 gebildet, die zwischen einem ersten Ankerabschnitt 50 und einem zweiten Ankerabschnitt 52 wirken. Hierzu ist an dem ersten Ankerabschnitt 50 beispielhaft ein elastisch verformbarer Federarm 51 ausgebildet, der einen Anschlagsnocken 53 beaufschlagt, der positionsstabil am zweiten Ankerabschnitt 52 gehalten ist. In einer eingeklappten Stellung des Ankers 4, 6, gemäß Fig. 3, drückt der Federarm 51 gegen diesen Anschlagsnocken 53 und erzeugt dadurch ein Drehmoment zwischen beiden Ankerabschnitten 50, 52, durch das diese in eine aufgeklappte Stellung vorgespannt sind. Der Anschlagsnocken 53 ist dabei, wie in Fig. 4 beispielhaft dargestellt, an einem Verriegelungsabschnitt 55 ausgebildet, der zur Sicherung beider Ankerabschnitte 50, 52 im zusammen gesteckten Zustand dient und der eine leichtere Montage des jeweiligen Ankers 4, 6 trotz dessen geringer Abmessungen ermöglicht.

Um dabei eine besonders sichere Lage der Anker 4, 6 an der ersten Seite 32 des Gewebes G gewährleisten zu können, können diese beispielsweise zumindest in einem Kontaktbereich 48 mit einer rauen Oberfläche versehen sein, wie in Fig. 2 dargestellt.

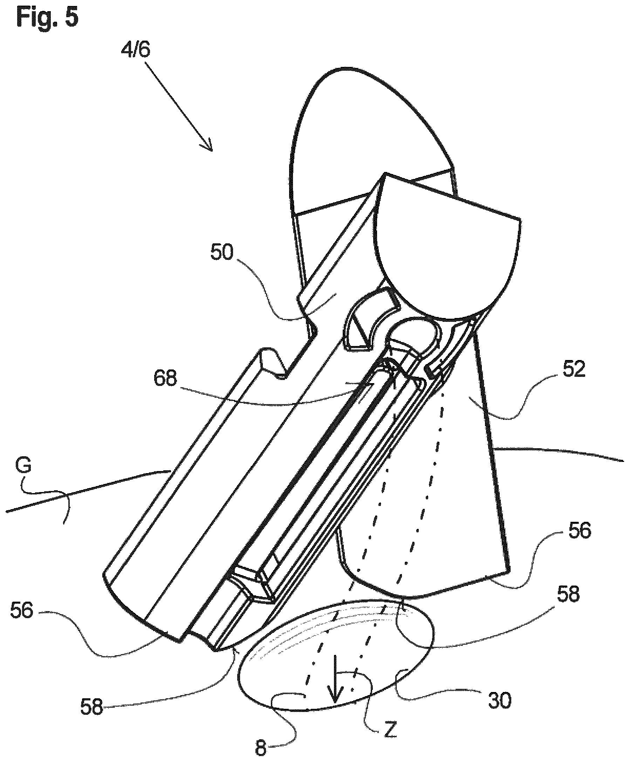

Wie aus Fig. 5 zu entnehmen ist, wird dieser Anker 4, 6 nach dem Setzen aus der Hohlnadel 14 heraus, durch die Federeinrichtung 68 zumindest soweit aufgeklappt, dass ein Wiedereintritt in die Durchtrittsöffnung 30 nicht möglich ist. Dabei weisen die beiden Ankerabschnitte 50, 52 an ihrem jeweiligen freien Ende 56 eine Abrundung 58 auf, wodurch sie durch Aufbringung der Zugkraft Z am Nahtelement 8 derart gegen das Gewebe G angepresst werden können, dass sie weiter in die vollständige Aufklappstellung verlagert werden können. Alternativ hierzu kann die Federeinrichtung 68 auch so ausgelegt werden, dass das durch sie erzeugbare Drehmoment MD ausreicht, um den Anker 4, 6 nach dem Austritt aus der Hohlnadel 14 vollständig in die Aufklappstellung zu verbringen.

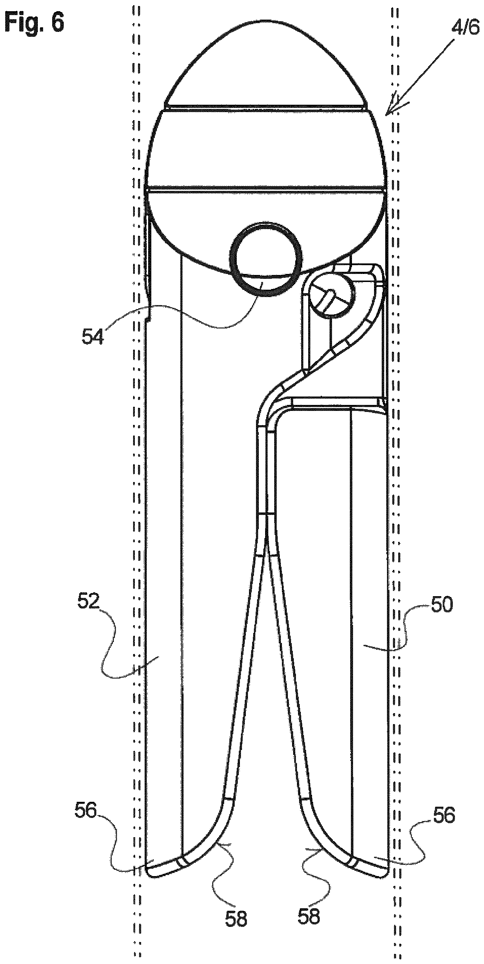

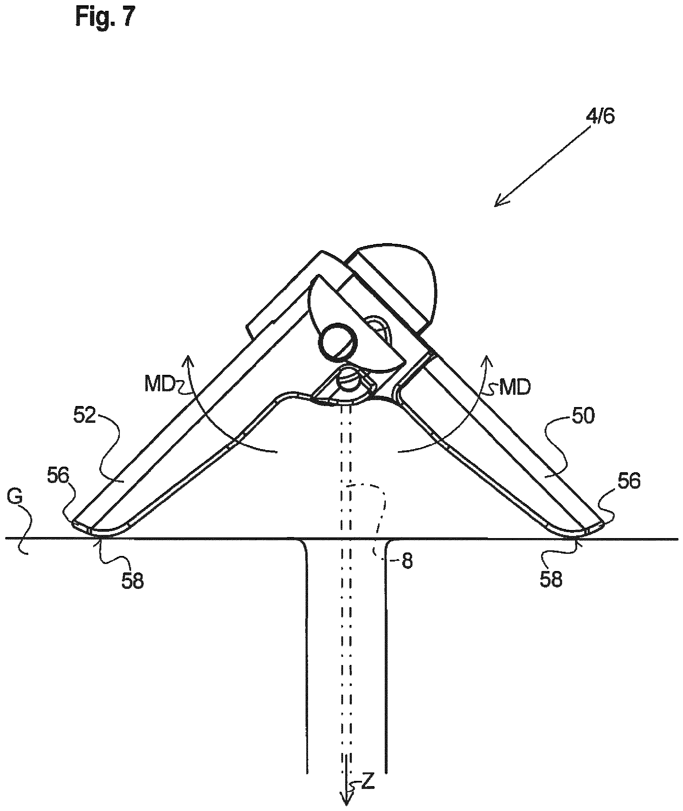

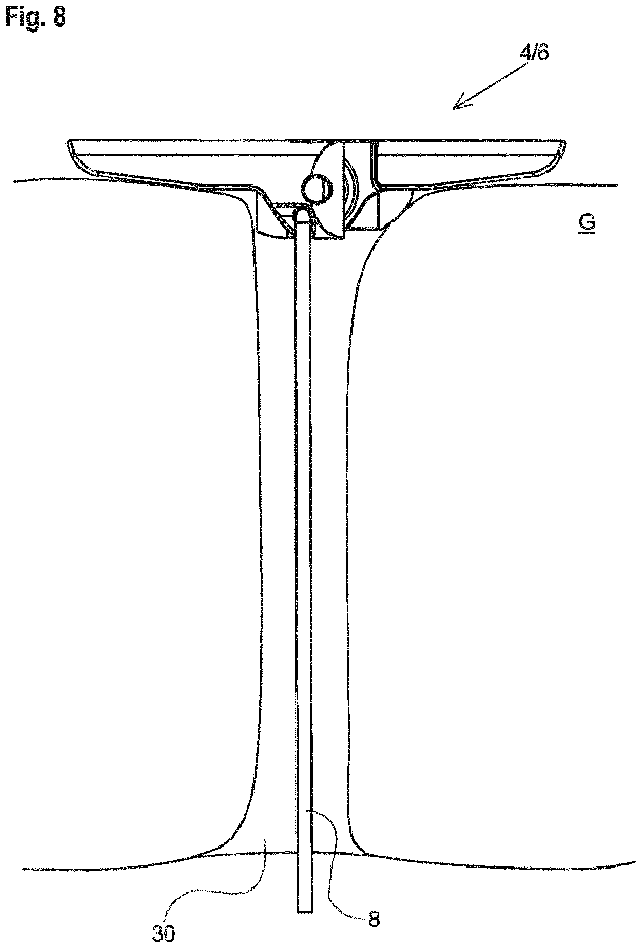

Wie beispielhaft in einer weiteren Ausführungsform nach Fig. 6 dargestellt, sind die beiden Ankerabschnitte 50, 52 zunächst in einer Einklappstellung angeordnet, in der sie in der Hohlnadel 14 aufgenommen und entlang derselben verlagert werden können. Beide Ankerabschnitte 50, 52 können dabei an einem jeweiligen proximalen freien Ende 56 eine Abrundung 58 aufweisen, so dass sie nach dem Setzen beispielsweise durch Anpressen der freien Enden 56 gegen das betreffende Gewebe G, insbesondere zusätzlich zu der je nach Ausführungsform vorgesehenen Federeinrichtung 68, voneinander weg verschwenkt werden können, wie in Fig. 7 dargestellt. Durch weiteren Zug am Nahtelement 8 können die beiden Ankerabschnitte 50, 52 dabei in die Aufklappstellung gemäß Fig. 8 geklappt werden, in der die Anker 4, 6 eine maximale Erstreckung aufweisen, die einen Wiedereintritt in die jeweilige Durchtrittsöffnung 30 verhindert. Zudem können am Anker 4, 6 auch Mittel vorgesehen sein, durch die die Ankerabschnitte 50, 52 in der Aufklappstellung gegenseitig verriegelt werden können (nicht dargestellt).

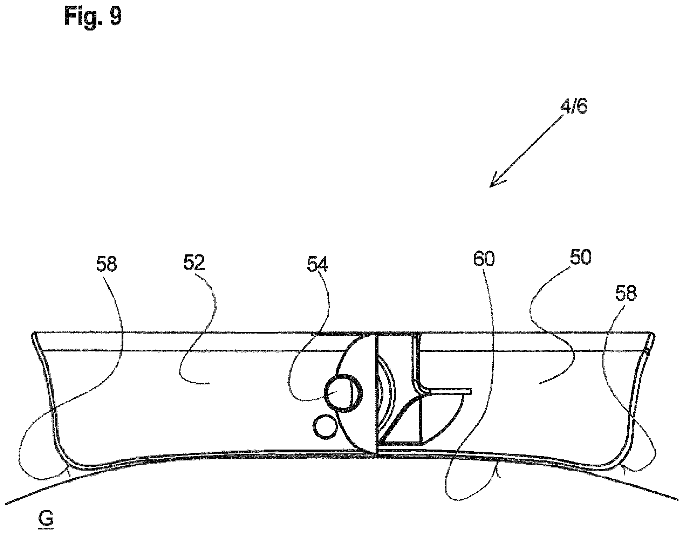

Wie in der Ausführungsform nach Fig. 9 dargestellt, ist es auch möglich, die beiden Ankerabschnitte 50, 52 derart auszuformen, dass sie in der Aufklappstellung eine gemeinsame konkave Kontaktfläche 60 bilden, die auf eine Krümmung einer konvexen Oberfläche des jeweils zu reparierenden Gewebes G abgestimmt ist, um eine besonders stabile Festlegung der Anker 4, 6 zu gewährleisten.

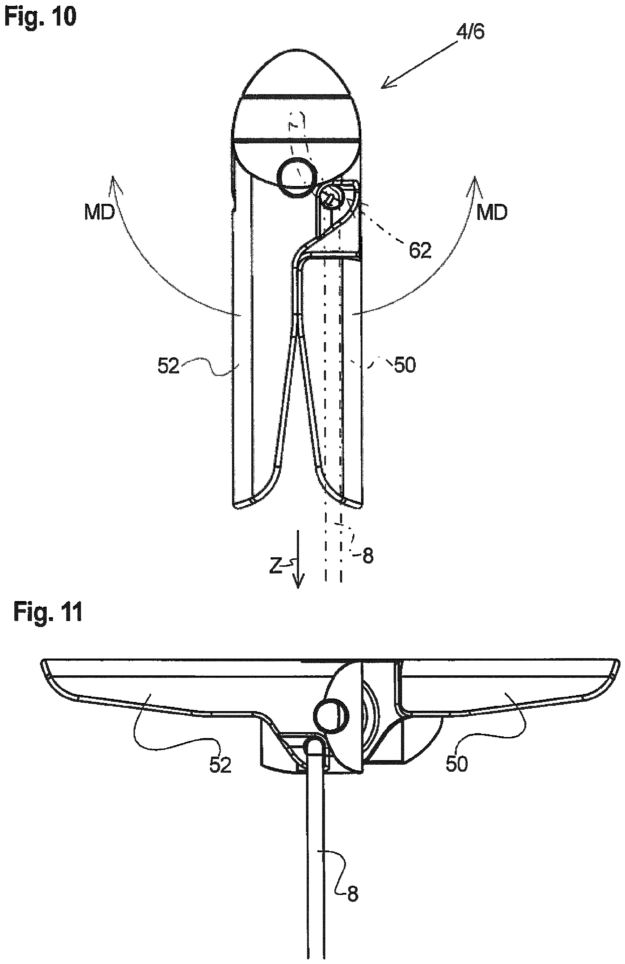

Fig. 10 zeigt eine weitere Ausführungsform der Anker 4, 6 in der Einklappstellung, bei der das Nahtelement 8 derart durch eine Anordnung von Aufnahmeöffnungen 62 beider Ankerabschnitte 50, 52 durch geschleift ist, dass die Aufbringung der Zugkraft Z am Nahtelement 8 eine Verkürzung des Nahtelementes 8 innerhalb des Ankers 4, 6 bewirkt. Diese Verkürzung hat wiederum die Beaufschlagung der beiden Ankerabschnitte 50, 52 mit entgegen gesetzt gerichteten Drehmomenten MD zur Folge, so dass diese voneinander weg geklappt werden, wie in Fig. 11 dargestellt.

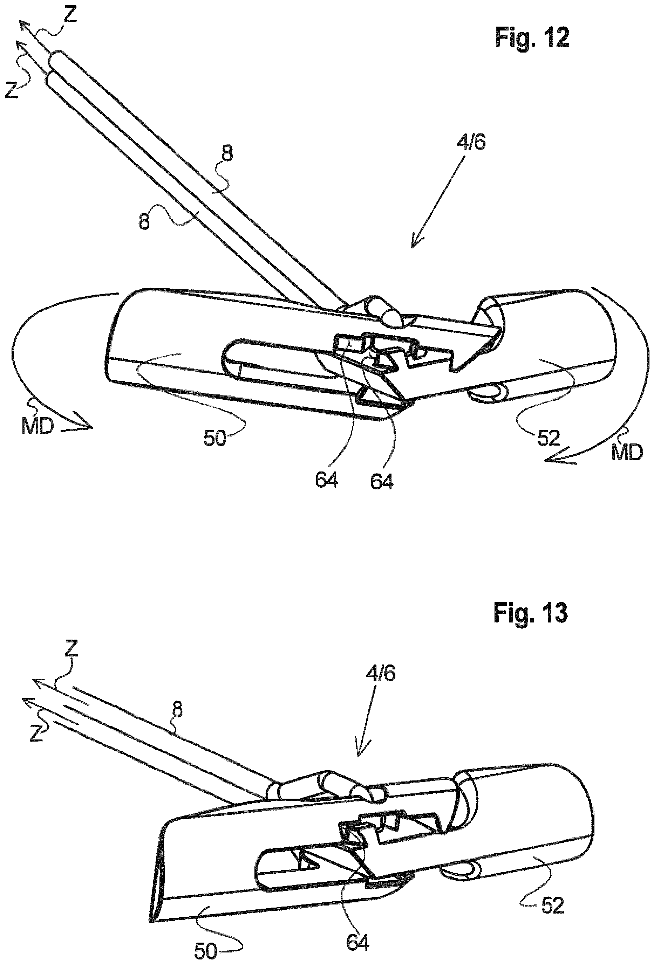

Wie aus Fig. 12 zu entnehmen ist, können an beiden Ankerabschnitten 50, 52 Anschlagsflächen 64 ausgebildet sein, die beim Aufklappen des jeweiligen Ankers 4, 6 auf eine jeweils entsprechende Anschlagsfläche 64 des jeweils anderen Ankerabschnittes 52, 50 zubewegt werden, wie aus Fig. 13 zu entnehmen ist. In der endgültigen Aufklappstellung liegen diese Anschlagsflächen 64 dann aneinander an und bilden somit einen Endanschlag für den jeweils anderen Ankerabschnitt 50, 52.

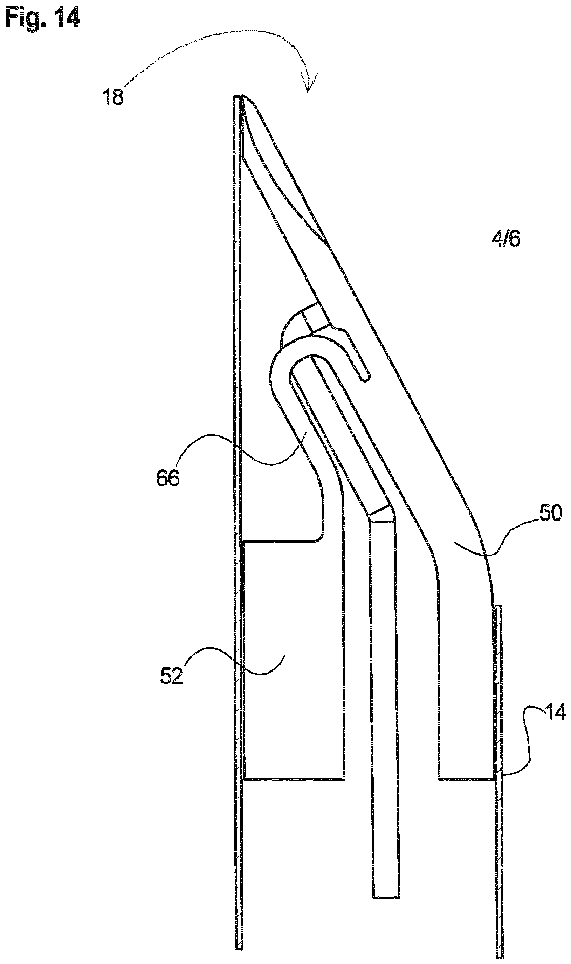

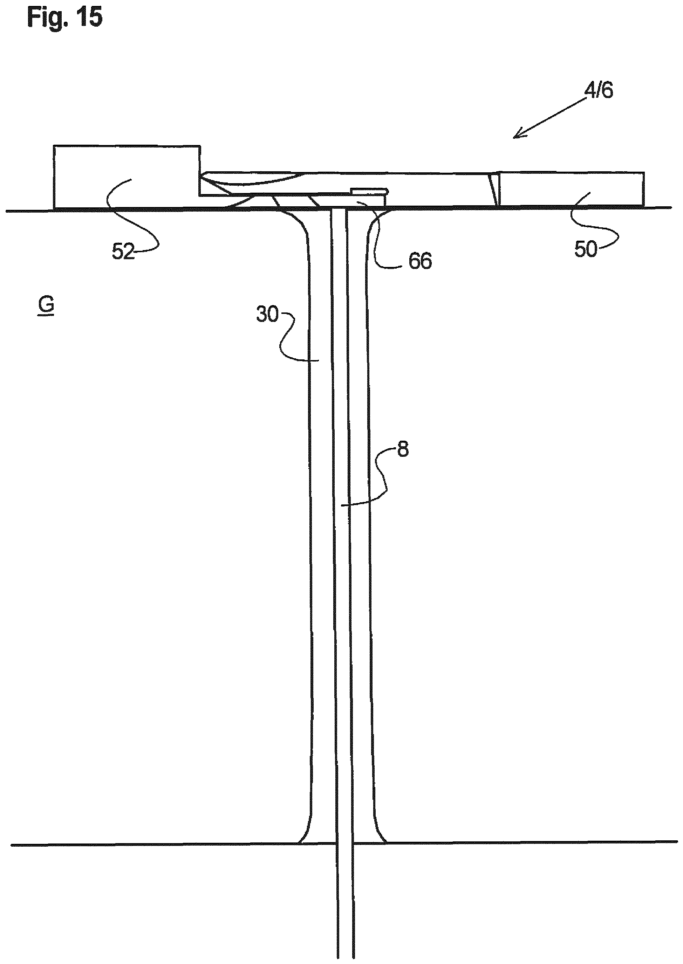

Fig. 14 zeigt eine weitere Ausführungsform der Anker 4, 6, bei der beide Ankerabschnitte 50, 52 einteilig ausgebildet und über ein Filmscharnier 66 zueinander verschwenkbar sind. In der gezeigten Einklappstellung sind die beiden Ankerabschnitte 50, 52 dabei derart zusammen geklappt, dass sie innerhalb der Hohlnadel 14 aufgenommen und verschoben werden können, wobei der erste Ankerabschnitt 50 die Nadelspitze 18 der Hohlnadel 14 verschließt. Nach dem Setzen des betreffenden Ankers 4, 6 erfolgt dann ein Umlenken in die Aufklappstellung gemäß Fig. 15, bei der beide Ankerabschnitte 50, 52 in einer Linie angeordnet sind und somit eine maximale Erstreckung aufweisen um positionsstabil am Gewebe G angelegt werden zu können.

Die Umlenkbewegung kann hierbei beispielsweise durch elastische Rückverformungskräfte des Filmscharniers 66 erfolgen. Hierzu muss das Filmscharnier 66 durch ein entsprechend elastisch verformbares Material gebildet sein und unter Vorspannung in Richtung der Aufklappstellung in die Hohlnadel 14 verbracht werden.

Alternativ oder zusätzlich hierzu ist es auch möglich zumindest Teile des Ankers 4, 6 beziehungsweise des Filmscharniers 66 aus einem Formgedächtnismaterial herzustellen, das beispielsweise durch Beaufschlagung mit Licht, Temperatur oder einem elektrischen oder magnetischen Feld aktiviert werden kann, um den betreffenden Anker 4, 6 nach dem Setzen von der Einklappstellung in die Aufklappstellung rückzuverformen.

Beispielsweise kann eine solches Formgedächtnismaterial durch eine Temperatur in Höhe einer gewöhnlichen Körpertemperatur aktivierbar sein. Der betreffende Anker 4, 6 wird dann in die Einklappstellung verformt und auf einer niedrigeren Temperatur gehalten. Nach dem Setzen wird der Anker 4, 6 dann durch die Umgebungstemperatur im Körper auf einen entsprechenden Wert erwärmt und das Formgedächtnismaterial aktiviert, so dass ein Umlenken der Ankerabschnitte 50, 52 in die Aufklappstellung erfolgt. Zudem wäre es denkbar, dass der betreffende Anker wenigstens zwei aktivierbare Verformungsabschnitte aufweist, deren Formgedächtnismaterial derart ausgelegt ist, dass diese in einem zeitlichen Abstand umgelenkt werden (nicht dargestellt). Auf diese Weise kann beispielsweise durch die erste Verformung sichergestellt werden, dass der betreffende Anker 4, 6 unmittelbar nach dem Setzen nicht wieder in die Durchtrittsöffnung 30 eintritt, während mittels der zweiten Verformung eine gewünschte endgültige Form des Ankers 4, 6 hergestellt wird, in der er dann dauerhaft im Körper verbleibt.

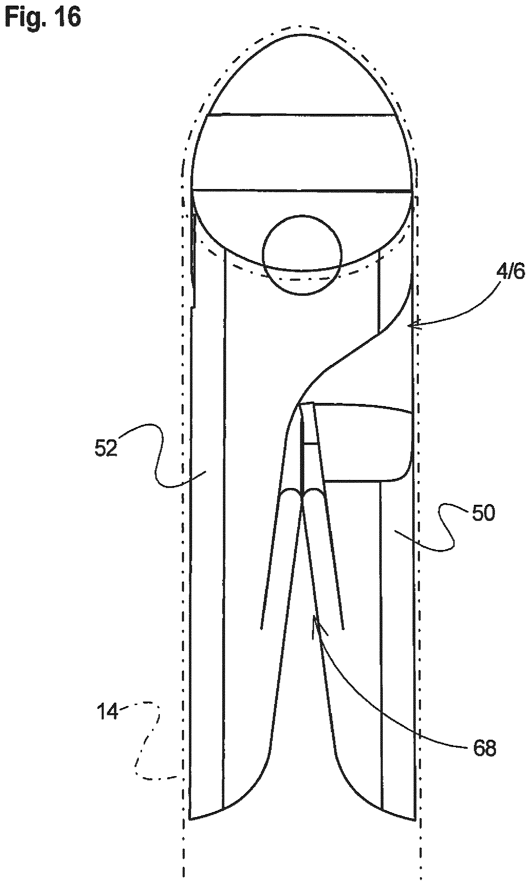

Fig. 16 zeigt eine weitere Ausführungsform eines Ankers 4, 6 mit wenigstens zwei zueinander verlagerbaren Ankerabschnitten 50, 52 mit der dazwischen angeordneten Federeinrichtung 68, mittels der die Ankerabschnitte 50, 52 in der in der Hohlnadel 14 eingenommenen Einklappstellung voneinander weg vorgespannt werden können.

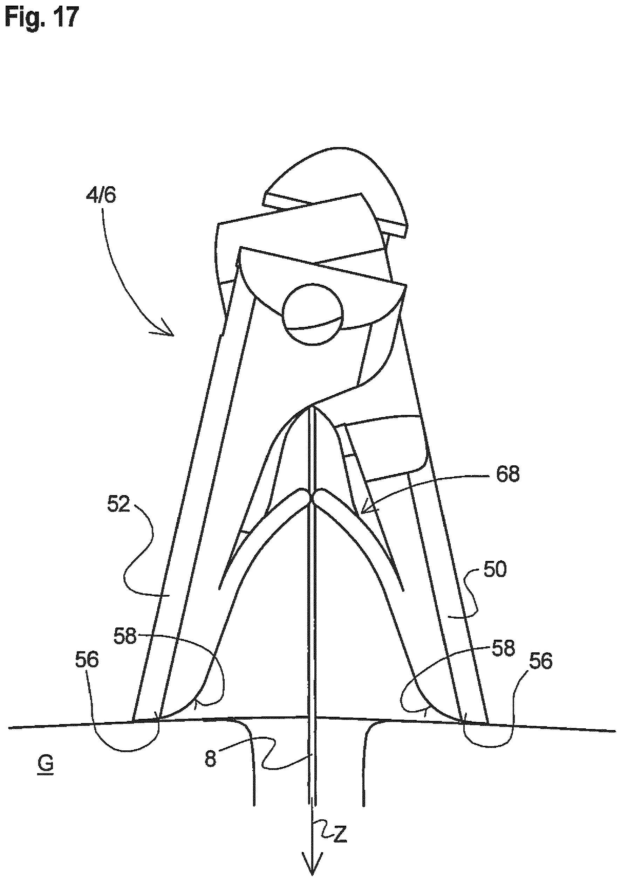

Wie aus Fig. 17 zu entnehmen ist, wird auch dieser Anker 4, 6 nach dem Setzen aus der Hohlnadel 14 heraus, durch die Federeinrichtung 68 zumindest soweit aufgeklappt, dass ein Wiedereintritt in die Durchtrittsöffnung 30 nicht möglich ist. Dabei weisen die beiden Ankerabschnitte 50, 52 an ihrem jeweiligen freien Ende 56 auch in dieser Ausführungsform die Abrundung 58 auf, wodurch sie durch Aufbringung der Zugkraft Z am Nahtelement 8 derart gegen das Gewebe G angepresst werden können, dass sie weiter in die vollständige Aufklappstellung verlagert werden können.

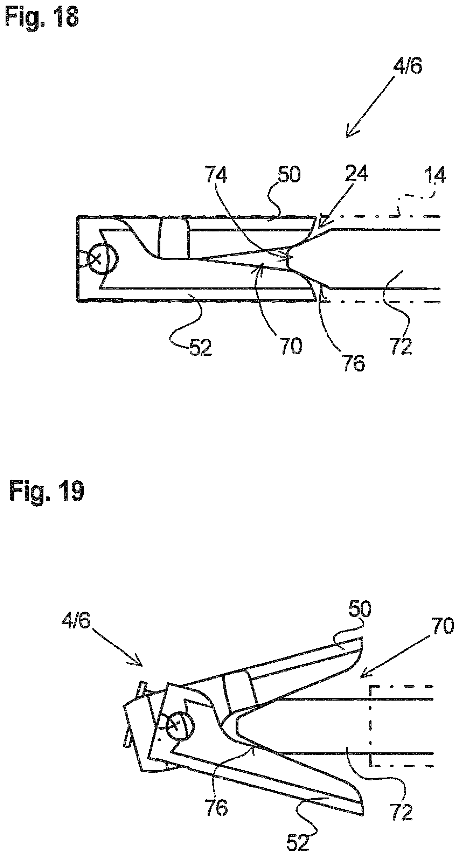

In einer weiteren Ausführungsform der Anker 4, 6, gemäß Fig. 18 bilden die Ankerabschnitte 50, 52 in der Einklappstellung beziehungsweise in dem in der Hohlnadel 14 aufgenommenen Zustand am proximalen Ende 24 des betreffenden Ankers 4, 6 zwischen sich eine gemeinsame Spreizaufnahme 70 aus. In diese Spreizaufnahme 70 können Aufspreizmittel 72 der Auswurfeinrichtung 46 angelegt werden, die an seinem distalen Ende 74 beispielsweise einen Verjüngungsbereich 76 ausformen. Dabei dienen die Aufspreizmittel 72 dazu, die beiden Ankerabschnitte 50, 52 nach Austritt des Ankers 4, 6 aus der Hohlnadel 14 durch eine Art Keilwirkung auseinanderzuklappen, wie in Fig. 19 dargestellt. Eine derartige Spreizaufnahme 70 kann dabei auch an jeder anderen Ausführungsform der Anker 4, 6 vorgesehen sein.

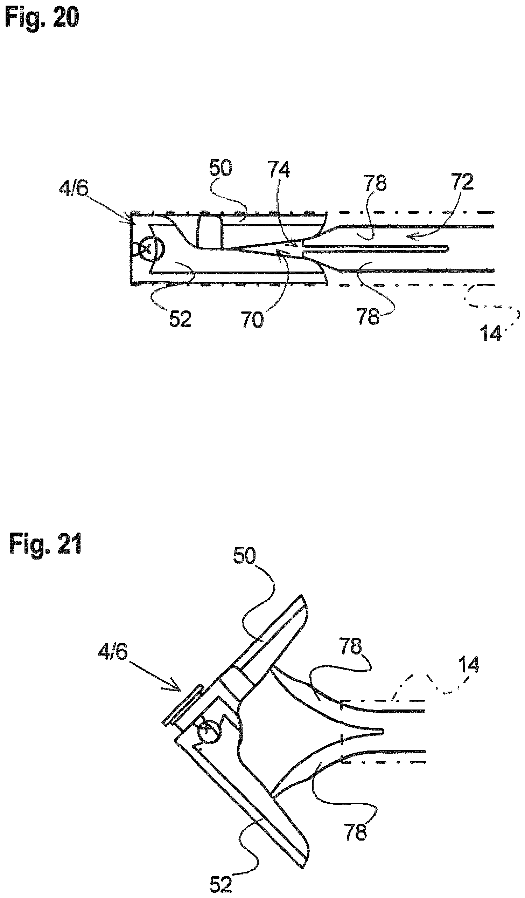

Fig. 20 zeigt eine alternative Ausführungsform der Aufspreizmittel 72, bei der am distalen Ende 74 wenigstens zwei elastisch radial nach außen vorgespannte Spreizarme 78 vorgesehen sind. Diese elastischen Spreizarme 78 beaufschlagen die Spreizaufnahme 70 des in der Einklappstellung in der Hohlnadel 14 aufgenommenen Ankers 4, 6. Nach dem Austritt dieses Ankers 4, 6 werden dessen beide Ankerabschnitte 50, 52 durch die elastischen Rückstellkräfte der Spreizarme 78 auseinandergeklappt, wie in Fig. 21 dargestellt.

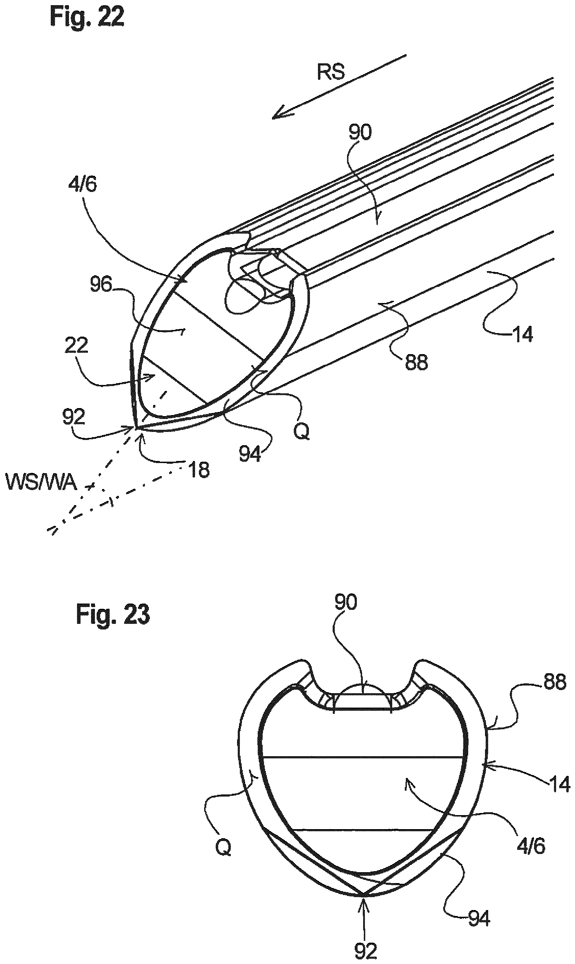

Wie aus Fig. 22 und 23 zu entnehmen ist, weist die Hohlnadel 14, über die die Anker 4, 6 gesetzt werden, zumindest an der Nadelspitze 18 einen geschlossen umlaufenden Querschnitt Q auf. Dabei ist der Querschnitt Q, wie dargestellt, stofflich geschlossen ausgebildet. Zudem ist die Hohlnadel 14 komplett geschlossen ausgebildet, wobei an ihrer Außenseite 88 eine Längsnut 90 eingelassen ist, die während eines Setzvorganges zur wenigstens teilweisen Aufnahme des Nahtelementes 8 dient.

Wie aus Fig. 22 und 23 ferner zu entnehmen ist, weicht der Querschnitt Q der Hohlnadel 14 wenigstens zur Nadelspitze 18 hin von einem Kreisprofil ab. Vielmehr verjüngt er sich herzförmig zu einer Querschnittsunterseite 92 hin. Zudem fällt ein Spitzenrand 94 der Nadelspitze 18 bezüglich der durch die Längserstreckung der Hohlnadel 14 zur Nadelspitze 18 hin definierte Setzrichtung RS schräg zu dieser Querschnittsunterseite 92 hin ab. Der Spitzenrand 94 weist dabei einen Anstellwinkel WS auf, der mit einem Anstellwinkel WA übereinstimmt, den eine Schräge 96 am distalen Ende 22 der Anker 4, 6 gegenüber der Längsachse des Grundkörpers 20 ausbildet. Hierdurch und durch einen angepassten Querschnitt der Anker 4, 6 können diese die Nadelspitze 18 im Wesentlichen bündig und dicht verschließen. Zudem sind die beiden Anker 4, 6 durch das entsprechend herzförmig ausgebildete Profil ihrer Führungsfläche 26 in einer vorgegebenen Drehstellung gegenüber der Hohlnadel 14 geführt.

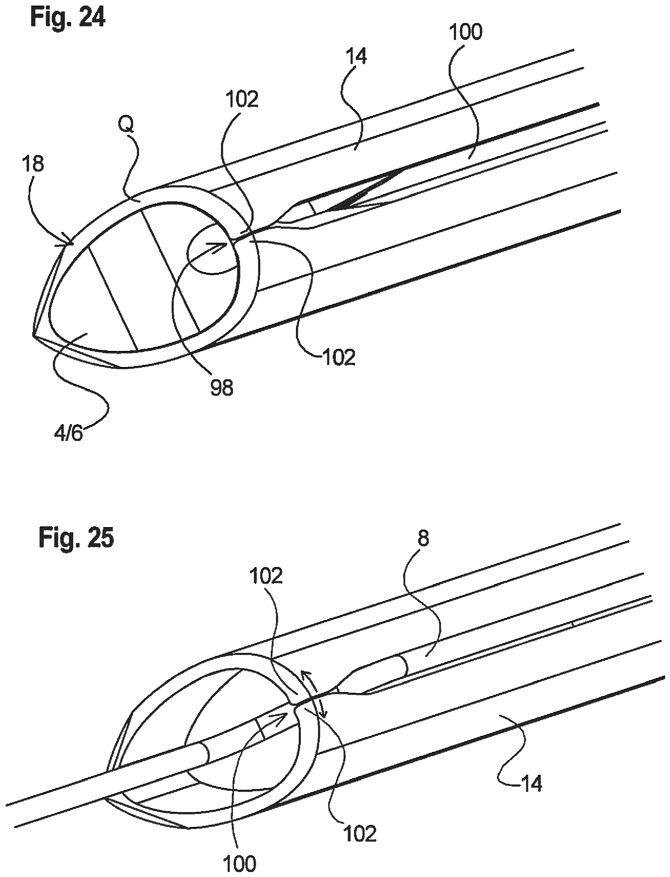

Fig. 24 zeigt eine alternative Ausführungsform der Hohlnadel 14, bei der der geschlossen umlaufende Querschnitt Q der Nadelspitze 18 einen Stoß 98 zweier Ränder 102 eines Längsschlitzes 100 aufweist. Dabei liegen die beiden Ränder 102 im unbelasteten Zustand zumindest an der Nadelspitze 18 aneinander. Beim Setzen der Anker 4, 6 kann der Längsschlitz 100 jedoch, wie in Fig. 25 dargestellt, durch Einpressen eines Abschnittes des Nahtelementes 8 oder eines abstehenden Teils des betreffenden Ankers 4, 6 (nicht dargestellt) zwischen die Ränder 102 geöffnet werden.

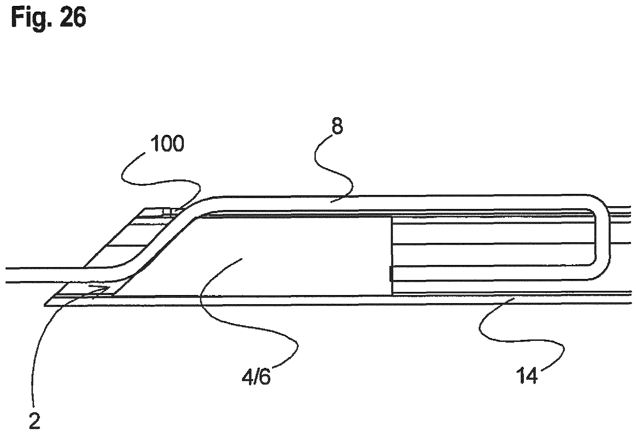

Alternativ hierzu kann der freie Querschnitt der Hohlnadel 14 zumindest abschnittsweise auch so eng dimensioniert werden, dass die Hohlnadel 14 allein durch die Verlagerung der Anker 4, 6 in distaler Richtung aufgespreizt und der Stoß 98 dabei geöffnet wird. Auf diese Weise kann insbesondere verhindert werden, dass das Nahtelement 8 beim Passieren des Stoßes 98 beschädigt oder gar durchtrennt wird. In jedem Fall kann hierbei ein von der Innenseite der Hohlnadel 14 durch den Längsschlitz 100 hindurch ragender Teil der Ankeranordnung 2, wie insbesondere das Nahtelement 8 gemäß Fig. 26, zusammen mit dem zweiten oder weiteren Anker 6 aus der Hohlnadel 14 heraus gesetzt werden.

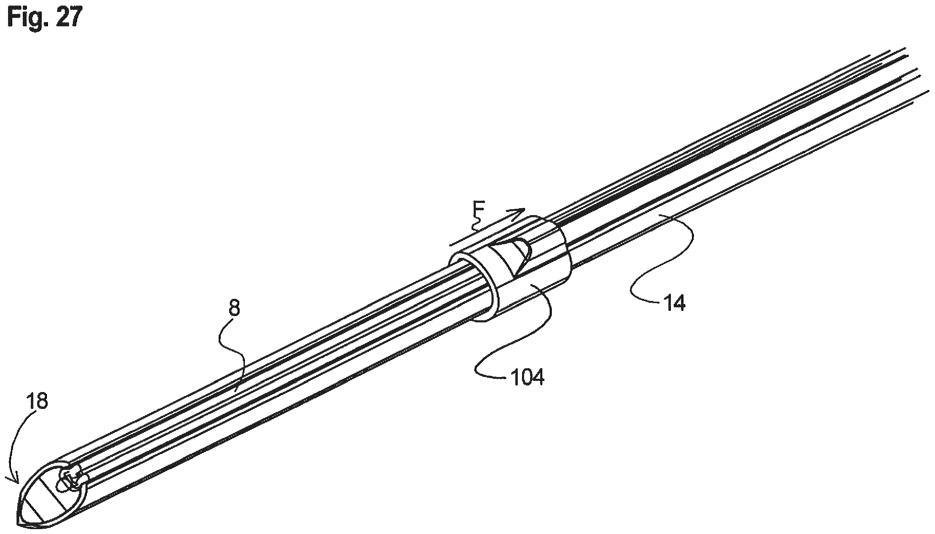

Um während eines Setzvorganges Behinderungen in Folge des Nahtelementes 8 verhindern zu können, können an der Hohlnadel 14 zudem Spannmittel vorgesehen sein, mittels denen das Nahtelement 8 benachbart zur Hohlnadel 14 gespannt und angelegt wird. Als Spannmittel kann hierbei, wie in Fig. 27 dargestellt, beispielsweise ein hülsenförmiges Schiebeelement 104 vorgesehen sein, das entlang der Hohlnadel 14 verschoben werden kann und an dem das Nahtelement umgelenkt ist. Zum Spannen des Nahtelementes 8 kann das Schiebeelement 104 dabei in proximaler Richtung durch eine Federkraft F beaufschlagt sein.

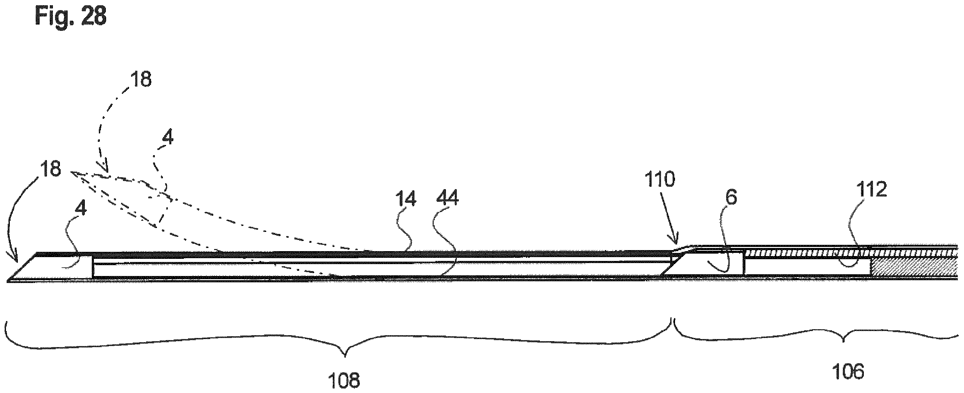

Um die wenigstens zwei Anker 4, 6 der Ankeranordnung 2 in einem nicht festgelegten Zeitabstand nacheinander aus der Hohlnadel 14 heraus setzen zu können, kann das Auswurfelement 44 beispielsweise jeweils direkt an dem in der setzbereiten Position angeordneten Anker 4, 6 angelegt werden, um diesen aus der Nadelspitze 18 heraus setzen zu können. Hierzu weist die Hohlnadel 14, wie in Fig. 28 dargestellt, einen Aufnahmeabschnitt 106 auf, der proximal zu einem distalen Abschnitt 108, vorgesehen ist. Dabei weist der Aufnahmeabschnitt 106 einen gegenüber dem distalen Abschnitt 108 erweiterten Querschnitt auf, so dass das Auswurfelement 44 an dem im Aufnahmeabschnitt 106 aufgenommenen zweiten Anker 6 vorbei geführt und direkt an dem ersten Anker 4 in der setzbereiten Position angelegt werden kann.

Wie aus Fig. 28 ferner zu entnehmen ist, ist der im Aufnahmeabschnitt 106 aufgenommene zweite Anker 6 dabei in Setzrichtung RS durch einen Querschnittssprung 110 der Hohlnadel 14 abgestützt. Zudem ist ein weiteres Stützmittel 112, beispielhaft in Form eines verlagerbaren Schiebers vorgesehen, das den zweiten Anker an seinem proximalen Ende 24 abstützt. Hierdurch ist der zweite Anker 6 in seiner im Aufnahmeabschnitt 106 aufgenommenen passiven Stellung in beiden axialen Richtungen bezüglich der Hohlnadel 14 stabil festgelegt.

Zum Setzen des ersten Ankers 4 wird dieser mittels des Auswurfelementes 44 in Setzrichtung RS verschoben, wobei das Auswurfelement 44 an dem im Aufnahmeabschnitt 106 festgelegten zweiten Anker 6 vorbei verlagert wird. Nach erfolgtem Auswurf des ersten Ankers 4 wird dann das Auswurfelement 44 in proximaler Richtung bis hinter den zweiten Anker 6 verlagert. Hierdurch wird ein seitliches Ausweichen des zweiten Ankers 6 und dessen Passieren des Querschnittssprunges 110 in den distalen Abschnitt 108 hinein ermöglicht, wie beispielsweise durch eine leicht federnde Beaufschlagung des proximalen Endes 24 durch das Stützmittel 112.

Hiernach kann nun das Auswurfelement 44 an das proximale Ende 24 angelegt werden, um den zweiten Anker 6 entlang dem distalen Abschnitt 108 zu verlagem, bis der zweite Anker 6 in der setzbereiten Position angeordnet ist, in der er die Nadelspitze 18 verschließt.

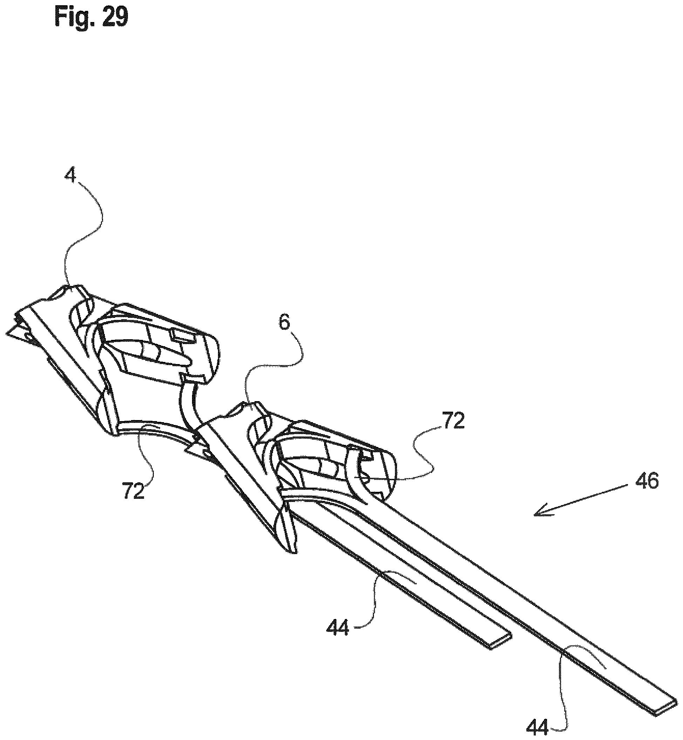

Alternativ zu der vorgenannten Vorgehensweise kann eine Auswurfeinrichtung 46 vorgesehen werden, die für die wenigstens zwei zu setzenden Anker 4, 6 jeweils ein eigenes Auswurfelement 44 aufweist. In Fig. 29 ist dieses Prinzip anhand zweier klappbarer Anker 4, 6 dargestellt, bei denen die Auswurfelemente 44 mit Aufspreizmitteln 72 versehen sind. Die Verwendung von wenigstens zwei separaten Auswurfetementen 44 ist jedoch auch bei jeder anderen der oben beschriebenen Ausführungsformen der Anker 4, 6 möglich.

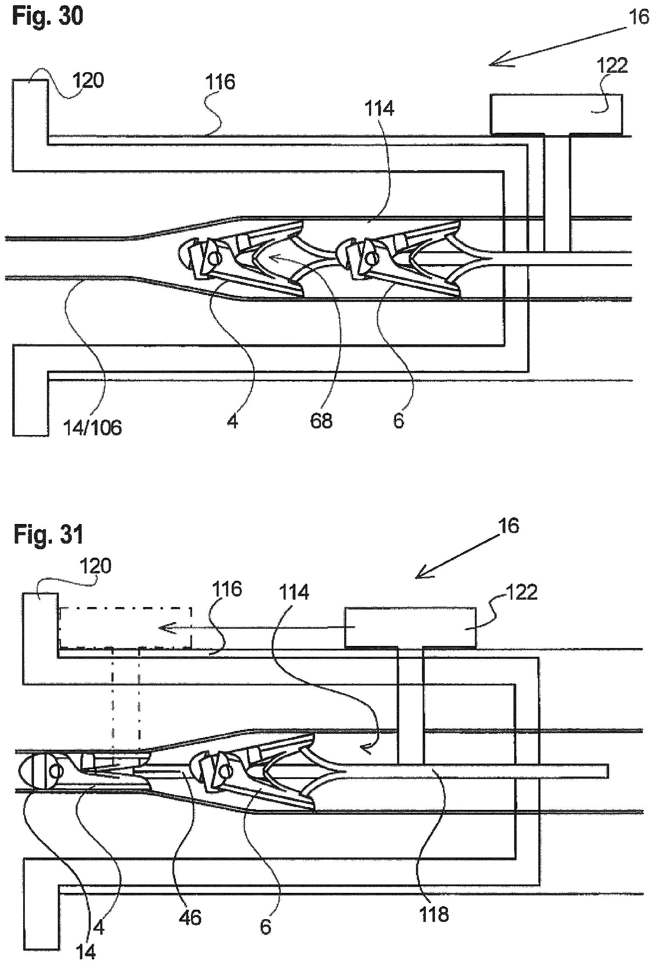

Darüber hinaus kann die Hohlnadel 14, wie in Fig. 30 dargestellt, auch mit einer Griffaufnahme 114 verbunden sein, die in einem Instrumentengriff 116 des chirurgischen Instrumentes 16 untergebracht ist. In dieser Griffaufnahme 114, die einen gegenüber dem distalen Abschnitt 108 (siehe Fig. 28) und auch gegenüber dem proximalen Aufnahmeabschnitt 106 der Hohlnadel 14 deutlich erweiterten freien Querschnitt aufweist, kann wenigstens einer der Anker 4, 6 oder können auch zwei oder mehrere Anker 4, 6 aufgenommen werden, bevor sie zur Vorbereitung eines Setzvorganges in die passive Stellung oder in die setzbereite Position innerhalb der Hohlnadel 14 verlagert werden.

Wie aus Fig. 30 zu entnehmen ist, können die in der Griffaufnahme 114 aufgenommenen Anker 4, 6 dabei in einer spannungsfreien Stellung, wie beispielsweise einer teilweise aufgeklappten Stellung aufgenommen werden. Hierdurch können insbesondere elastische Federeinrichtung 68 von aufklappbaren Ankern 4, 6 während der Speicherung in der Griffaufnahme 114 entlastet werden, um eine Abnahme der mit der Federeinrichtung 68 erzeugbaren elastischen Rückstellkräfte vermeiden zu können.

Aus der Griffaufnahme 114 können die Anker 4, 6 zur Vorbereitung eines Setzvorganges in den distalen Abschnitt 108 oder, sofern vorgesehen auch in den proximalen Aufnahmeabschnitt 106 der Hohlnadel 14 verlagert werden, wie in Fig. 31 dargestellt. Hierzu weist die Auswurfeinrichtung 46 beispielsweise einen vom Benutzer betätigbaren Schieber 118 auf, mittels dem der betreffende Anker 4, 6 in die gewünschte Position beziehungsweise in die setzbereite Position in der Hohlnadel 14 verlagert werden kann. In der dargestellten Ausführungsform des ersten Ankers 4 als klappbarer Anker wird dieser dabei gleichzeitig in die Einklappstellung verbracht. Der Schieber 118 kann dabei beispielsweise über ein Bedienelement 122 betätigt werden, das selbst beweglich am übrigen Instrumentengriff 116 gelagert ist. Dabei ist das Bedienelement 122 in distaler Richtung bis zu einer mit strichpunktierten Linien dargestellten Stellung verlagerbar, die der setzbereiten Position des betreffenden Ankers 4, 6 entspricht.

Je nach Ausführungsform der Auswurfeinrichtung 46 kann der Schieber 118 hierbei selbst auch als Auswurfelement 46 fungieren oder, wie dargestellt getrennt zu einem separaten Auswurfelement 46 ausgeführt sein. In beiden Fällen kann der Auswurf dabei durch Verlagerung eines Betätigungselementes 120 erfolgen, das beispielhaft als verschiebbare Hülse dargestellt ist.

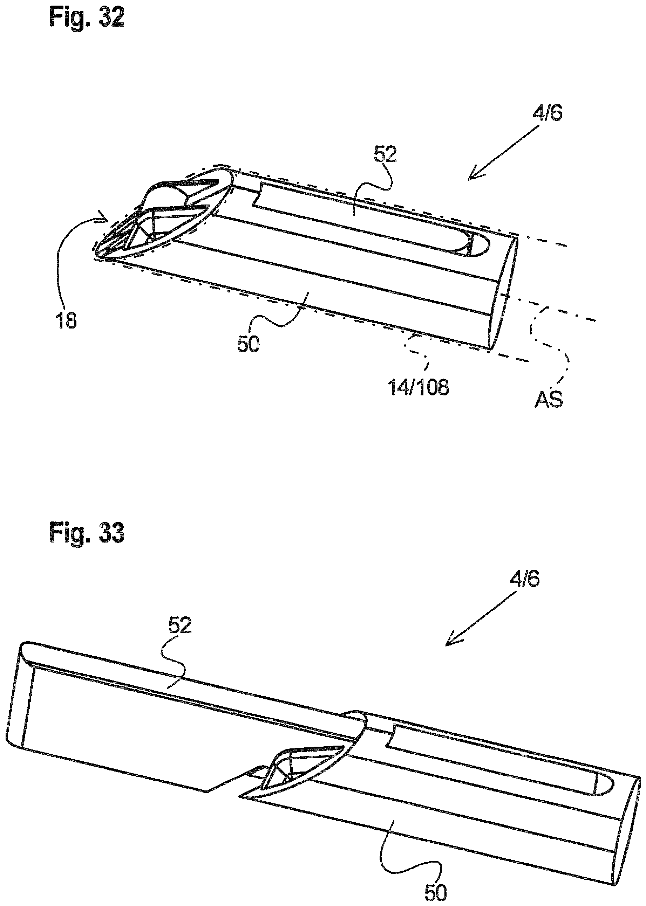

Es sei noch darauf hingewiesen, dass alternativ zu den beispielhaft dargestellten verschiedenen Ausführungsformen aufklappbarer Anker 4, 6, die bei unten angeordneter Nadelspitze 18 jeweils eine horizontale Klappbewegung vorsehen, die klappbaren Anker 4, 6 je nach vorgesehenem Anwendungsfall auch in jeder anderen Winkelstellung gegenüber einer durch den distalen Abschnitt 108 der Hohlnadel 14 definierten Setzachse AS aufklappbar sein können. Die Fig. 32 und 33 zeigen hierbei eine beispielhafte Ausführungsform eines klappbaren Ankers 4, 6, der bei unten angeordneter Nadelspitze 18 ein vertikales Aufklappen der beiden Ankerabschnitte 50, 52 vorsieht.

Darüber hinaus sei zudem darauf hingewiesen, dass die Hohlnadel 14 an ihrem distalen Ende 12 je nach vorgesehenem Anwendungsfall auch eine Krümmung aufweisen kann, wie in Fig. 28 durch strichpunktierte Linien dargestellt.

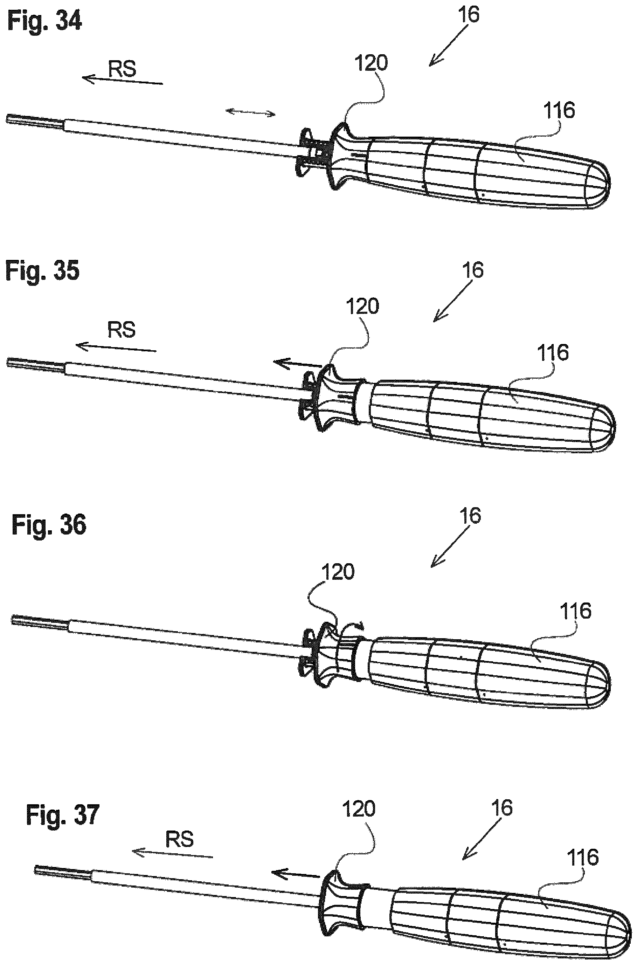

Wie bereits oben beschrieben, kann das chirurgische Instrument 16 beispielhaft als manuell betätigbares Gerät ausgebildet sein. Hierzu weist das chirurgische Instrument 16 das Betätigungselement 120 auf, das in einer aus Fig. 34 zu entnehmenden Ausgangstellung benachbart zum Instrumentengriff 116 angeordnet ist und gegenüber diesem parallel zur Setzrichtung RS verschoben werden kann.

Zur Verlagerung eines der Anker 4, 6 in die setzbereite Stellung an der Nadelspitze 18 wird das Betätigungselement 120 in die in Fig. 35 dargestellte Stellung verbracht, in der sie gegenüber dem übrigen Instrumentengriff 116 beabstandet ist.

Zum Setzen des betreffenden Ankers 4, 6 muss das Betätigungselement 120 dann wie in Fig. 36 dargestellt zunächst um seine Achse verdreht werden. Erst nach Erreichen einer vorbestimmten Drehstellung kann das Betätigungselement 120 gemäß Fig. 37 weiter in Setzrichtung RS verlagert werden, um den betreffenden Anker 4, 6 auszuwerfen. Nach diesem Setzvorgang erfolgt dann eine manuelle oder selbsttätige Rückverlagerung des Betätigungselementes 120 in die in Fig. 34 dargestellte Ausgangsstellung.

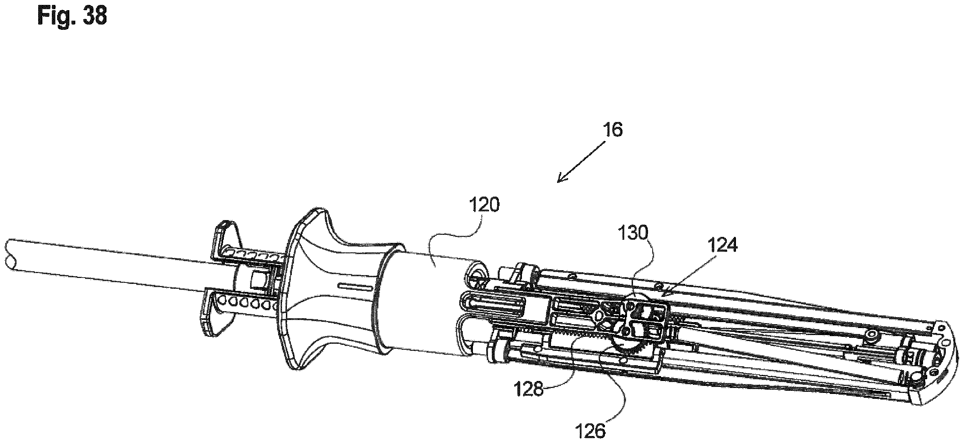

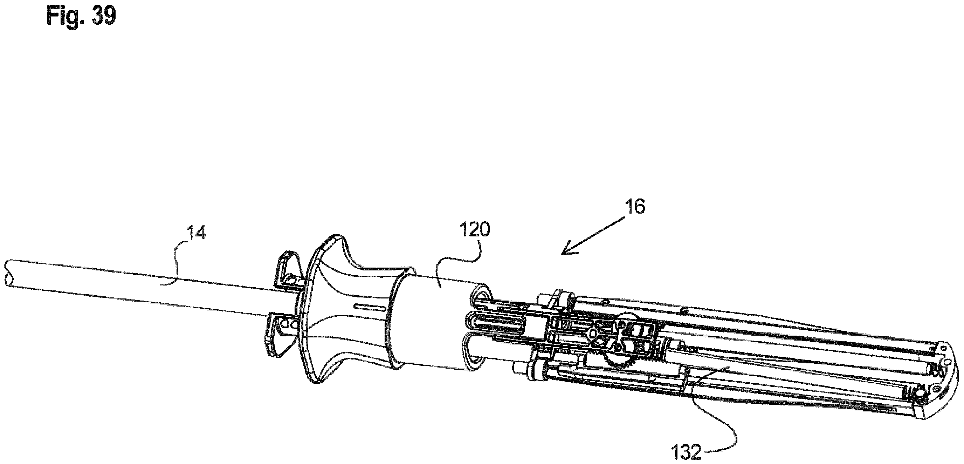

Wie aus Fig. 38 zu entnehmen ist, weist das chirurgische Instrument 16 hierbei ein Zahnradgetriebe 124 auf, das zur Steuerung der Bewegungsabläufe der Auswurfeinrichtung 46 dient. Das Zahnradgetriebe 124 weist dabei ein am Betätigungselement 120 gehaltenes mehrstufiges Zahnrad 126 auf, das sowohl mit einer gehäusefesten Verzahnung 128 des Instrumentengriffes 116 als auch mit einem Rad 130 kämmt, das mit der Auswurfeinrichtung 46 beispielsweise über ein Riemengetriebe gekoppelt ist (nicht dargestellt). Hierdurch kann die relative Bewegung des Betätigungselementes 120 gegenüber dem Instrumentengriff 116 mit einer bestimmten Übersetzung auf die Auswurfeinrichtung 46 übertragen werden, um beispielsweise mit einer relativ kleinen Verlagerung des Betätigungselementes 120 relativ große Verlagerungen der Auswurfeinrichtung 46 beziehungsweise des wenigstens einen Auswurfelementes 44 oder des wenigstens einen Schiebers 118 vornehmen zu können. Hierdurch ist es insbesondere möglich, den jeweiligen Anker 4, 6 von dessen Passivstellung im proximalen Aufnahmeabschnitt 106 durch eine geringe relative Verlagerung des Betätigungselementes 120 gegenüber dem Instrumentengriff 116 zu der in Fig. 39 dargestellte Stellung in die setzbereite Position in der Hohlnadel 14 zu verbringen.

Zudem ist es auch möglich, das Zahnradgetriebe 124 derart auszubilden, dass eine von mehreren Übersetzungen eingestellt werden kann (nicht dargestellt).

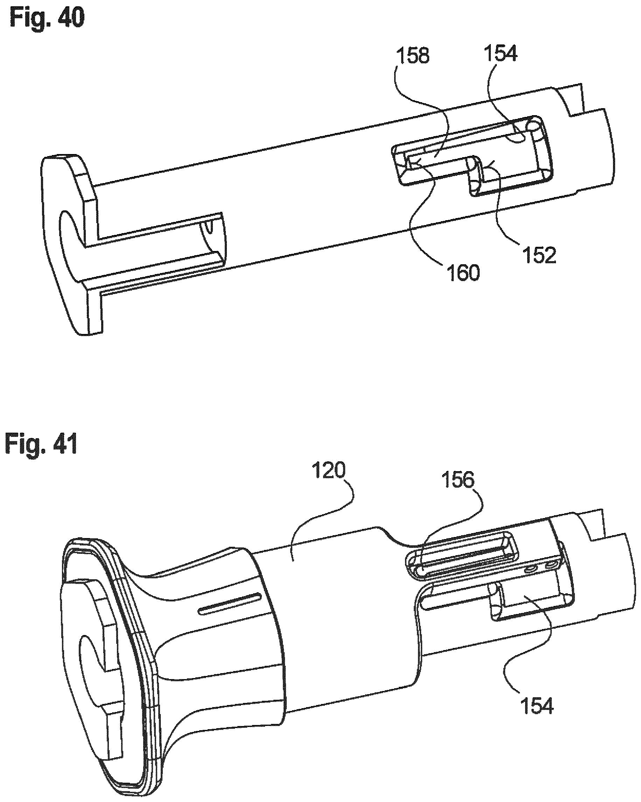

In der der setzbereiten Position des betreffenden Ankers 4, 6 entsprechenden Stellung des Betätigungselementes 120 beziehungsweise der Auswurfeinrichtung 46, gemäß Fig. 35 und 39, kommt das Betätigungselement 120 dabei in Setzrichtung RS in Anlage an einem Endanschlag 152. Wie insbesondere aus den Fig. 40 und 41 zu entnehmen ist, wird dieser Endanschlag 152 dabei durch eine am Instrumentengriff 116 vorgesehene Steuerkurve 154 gebildet, in die ein Nocken 156 hineinragt, der am Betätigungselement 120 mitgeführt ist.

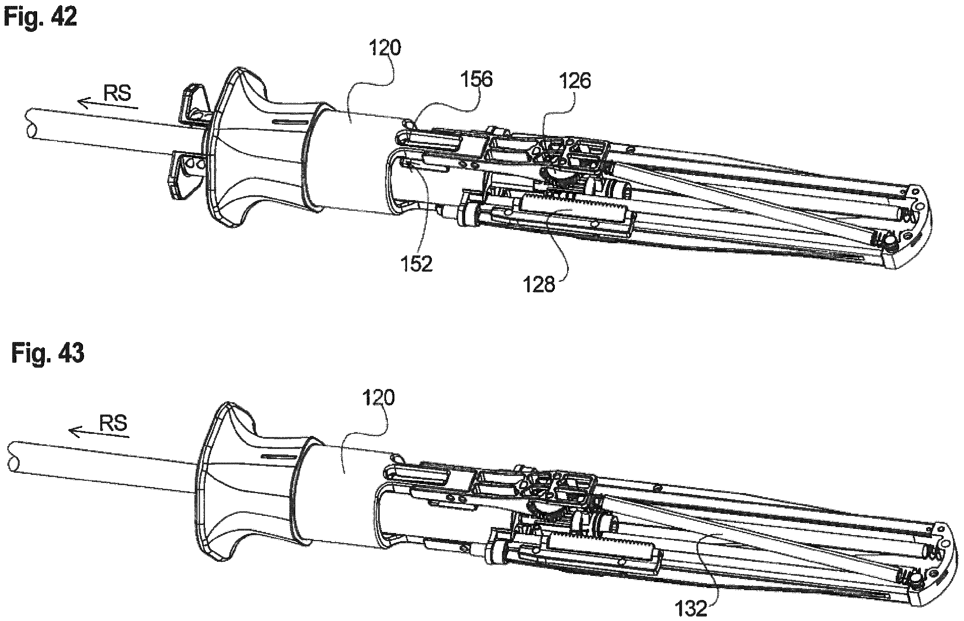

Aus dieser Endanschlagstellung kann der Nocken 156 durch die in Fig. 36 dargestellte Drehbewegung des Betätigungselementes 120 in eine in Fig. 42 dargestellte Freigabestellung verbracht werden, in der er bezüglich Setzrichtung RS seitlich beabstandet zum Endanschlag 152 angeordnet ist. Gleichzeitig ist in der Freigabestellung der kämmende Eingriff des Zahnrades 126 mit der gehäusefesten Verzahnung 128 aufgehoben.

Das Betätigungselement 120 mit dem Nocken 156 kann somit aus der Freigabestellung entlang eines Längsabschnittes 158 (siehe Fig. 40) der Steuerkurve 154 verlagert werden, wobei sich die Auswurfeinrichtung 46 weiter in Setzrichtung RS bewegt, um den betreffenden Anker 4, 6 auszuwerfen. Aufgrund der Trennung des Zahnrades 126 von der gehäusefesten Verzahnung 128 erfolgt hierbei keine Übersetzung, wodurch der Benutzer den Setzvorgang sehr direkt steuern kann.

Nach dem Setzen des betreffenden Ankers 4, 6 kommt der Nocken 156 in der in Fig. 43 dargestellten Position in Anlage an einem Auswurfendanschlag 160 der Steuerkurve 154 (siehe Fig. 40). In dieser Position ist eine weitere Verlagerung des Betätigungselementes 120 und der Auswurfeinrichtung 46 in Setzrichtung RS blockiert, um beispielsweise einen Austritt beziehungsweise einen zu weiten Austritt des Auswurfelementes 44 an der Nadelspitze 18 zu verhindern.
. Von dieser Auswurfendanschlagstellung kann das Betätigungselement 120 zusammen mit der Auswurfeinrichtung 46 beispielsweise nach Betätigung eines Auslöseknopfes (nicht dargestellt) durch die Federkraft einer Rückholfedereinrichtung 132 wieder in die Ausgangsstellung gemäß Fig. 41 zurück verlagert wird. Hierbei kann beispielsweise das Auswurfelement 44 der Auswurfeinrichtung 46 wie oben beschrieben am proximalen Ende 24 des nachfolgend zu setzenden zweiten Ankers 6 positioniert werden. Anschließend kann dann das Betätigungselement 120 wieder in die Stellung gemäß Fig. 38 verlagert werden, um nun den zweiten Anker 6 in die setzbereite Position an der Nadelspitze 18 zu verbringen.

Hierbei kann beispielsweise ein akustisch, optisch oder taktil wahrnehmbarer Signalgeber (nicht dargestellt) vorgesehen sein, der selbsttätig aktiviert wird, sobald sich der betreffende zweite oder weitere Anker 6 in der setzbereiten Position befindet. Ferner kann die Auswurfeinrichtung 46 derart ausgebildet sein, dass sie in der setzbereiten Position eines der Anker 4, 6 beispielsweise Ober das Zahnradgetriebe 124 gegen eine Bewegung entgegen der Setzrichtung RS gesperrt ist, so dass das Auswurfelement 44 den betreffenden Anker 46 in proximaler Richtung abstützt.

## Patentansprüche

1. Ankeranordnung (2) zur chirurgischen Gewebereparatur, wie insbesondere zur Reparatur eines Meniskusrisses (R),
mit wenigstens einem ersten Anker (4) und einem zweiten Anker (6), die für die jeweilige Platzierung an einem zu reparierenden Gewebe (G) entlang einer Hohlnadel (14) verschiebbar und über ein Nahtelement (8) miteinander verbunden sind,
wobei der erste Anker (4) und der zweite Anker (6) sich jeweils zwischen einem distalen Ende (22) und einem proximalen Ende (24) erstrecken und an ihrer Außenseite eine Führungsfläche (26) für die wenigstens teilweise Anlage an einer Innenseite (28) einer Hohlnadel (14) bilden,
und an den Ankern (4, 6) Umlenkmittel (38) vorgesehen sind, über die die Anker (4, 6) beim oder nach dem Setzen wenigstens teilweise mit einem Drehmoment (MD) beaufschlagbar sind,
**dadurch gekennzeichnet, dass** wenigstens einer der Anker (4, 6) wenigstens zwei relativ zueinander verlagerbare Ankerabschnitte (50, 52) aufweist, die zwischen einer Einklappstellung, in der die Führungsfläche (26) einen von einem Kreisprofil abweichenden Querschnitt (Q) aufspannt, und einer Aufklappstellung relativ zueinander verschwenkbar sind und die wenigstens zwei Ankerabschnitte (50, 52) über ein Gelenk (54) miteinander verbunden sind.

2. Ankeranordnung nach Anspruch 1, **dadurch gekennzeichnet, dass** die Führungsfläche (26) in der Einklappstellung der zueinander verlagerbaren Ankerabschnitte (50, 52) ein herzförmiges, ovales oder eiförmiges im Sinne von umlaufend konvexes Profil ausbildet.

3. Ankeranordnung nach Anspruch 1 oder 2, **dadurch gekennzeichnet, dass** die wenigstens zwei Ankerabschnitte (50, 52) in Richtung der Aufklappstellung vorspannbar sind, wobei das Nahtelement (8) an den Ankerabschnitten (50, 52) derart umgelenkt ist, dass diese mit einem Aufklappmoment beaufschlagt werden können.

4. Ankeranordnung nach einem der Ansprüche 1 bis 3, **dadurch gekennzeichnet, dass** die wenigstens zwei Ankerabschnitte (50, 52) in der Aufklappstellung jeweils durch einen Endanschlag des jeweils anderen Ankerabschnittes (52, 50) abgestützt sind.

5. Ankeranordnung nach einem der Ansprüche 1 bis 4, **dadurch gekennzeichnet, dass** die wenigstens zwei Ankerabschnitte (50, 52) in der Einklappstellung am proximalen Ende (24) wenigstens eines der Anker (4, 6) eine gemeinsame Spreizaufnahme (70) bilden, die durch Beaufschlagung mittels eines Aufspreizmittels (72) aufspreizbar ist.

6. Chirurgisches Instrument (16) mit einer Ankeranordnung (2) nach einem der Ansprüche 1 bis 5, zur chirurgischen Gewebereparatur, wie insbesondere zur Reparatur eines Meniskusrisses (R),
mit einer Hohlnadel (14), die an einem distalen Ende eine Nadelspitze (18) ausbildet,
und einer Auswurfeinrichtung (46) mit einem innerhalb der Hohlnadel (14) verlagerbaren Auswurfelement (44), mittels dem der erste und zweite Anker (4, 6) entlang der Hohlnadel (14) verschiebbar sind,
**dadurch gekennzeichnet, dass** die Hohlnadel (14) an der Nadelspitze (18) einen geschlossen umlaufenden oder schließbaren Querschnitt (Q) aufweist und wenigstens zur Nadelspitze (18) hin einen von einem Kreisprofil abweichenden Querschnitt (Q) aufweist.

7. Chirurgisches Instrument nach Anspruch 6, **dadurch gekennzeichnet, dass** die Hohlnadel (14) einen Längsschlitz (100) aufweist und zwei sich gegenüberliegende Ränder (102) des Längsschlitzes (100) im unbelasteten Zustand an der Nadelspitze (18) aneinander liegen.

8. Chirurgisches Instrument nach Anspruch 7, **dadurch gekennzeichnet, dass** die Ränder (102) mittels Einpressen eines Abschnittes des Nahtelementes (8) oder der Anker (4, 6) aufweitbar sind.

9. Chirurgisches Instrument nach einem der Ansprüche 6 bis 8, **dadurch gekennzeichnet, dass** die Nadelspitze (18) durch den ersten und den zweiten Anker (4, 6) der darin aufgenommenen Ankeranordnung (2) jeweils bündig verschließbar ist.

10. Chirurgisches Instrument nach einem der Ansprüche 6 bis 9, **dadurch gekennzeichnet, dass** an der Hohlnadel (14) Spannmittel zum Spannen des Nahtelementes (8) vorgesehen sind, die ein entlang der Hohlnadel (14) verschiebbares Schiebeelement (104) aufweisen, an dem das Nahtelement (8) umgelenkt ist und das in proximaler Richtung vorgespannt ist.

11. Chirurgisches Instrument nach einem der Ansprüche 6 bis 10, **dadurch gekennzeichnet, dass** die Hohlnadel (14) an der Nadelspitze (18) einen distalen Abschnitt (108) und einen hierzu proximal angeordneten Aufnahmeabschnitt (106) aufweist, der gegenüber dem distalen Abschnitt (108) einen erweiterten Querschnitt aufweist.

12. Chirurgisches Instrument nach Anspruch 11, **dadurch gekennzeichnet, dass** in dem erweiterten Querschnitt das Auswurfelement (44) an dem zweiten Anker (6) vorbei führbar ist.

13. Chirurgisches Instrument nach Anspruch 11 oder 12, **dadurch gekennzeichnet, dass** in dem proximalen Aufnahmeabschnitt (106) Festlegemittel vorgesehen sind, mittels denen der zweite Anker (6) in beide axialen Richtungen abstützbar ist.

14. Chirurgisches Instrument nach einem der Ansprüche 6 bis 13, **dadurch gekennzeichnet, dass** ein Zahnradgetriebe (124) zur Steuerung der Bewegungsabläufe der Auswurfeinrichtung (46) vorgesehen ist und das Zahnradgetriebe (124) eine vorbestimmte Übersetzung zwischen einem verlagerbaren Betätigungselement (120) und dem Auswurfelement (44) aufweist.

15. Chirurgisches Instrument nach Anspruch 14, **dadurch gekennzeichnet, dass** die Anker (4, 6) in einer setzbereiten Position in proximaler Richtung durch die Auswurfeinrichtung (46) abstützbar sind und deren Bewegung in proximaler Richtung hierbei gesperrt ist.

16. Chirurgisches Instrument nach Anspruch 14 oder 15, **dadurch gekennzeichnet, dass** das Betätigungselement (120) an einen Endanschlag (152) anlegbar ist und die Auswurfeinrichtung (46) dabei in einer Endanschlagstellung angeordnet ist, die der setzbereiten Position des jeweils mitgeführten Ankers (4, 6) entspricht.

17. Chirurgisches Instrument nach Anspruch 16, **dadurch gekennzeichnet, dass** das Betätigungselement (120) unter Trennung des Zahnradgetriebes (124) von der Endanschlagstellung in eine Freigabestellung verdrehbar ist, in der das Betätigungselement (120) weiter in Setzrichtung (RS) verlagerbar ist.

18. Chirurgisches Instrument nach Anspruch 16 oder 17, **dadurch gekennzeichnet, dass** die Endanschlagstellung und die Freigabestellung durch eine Steuerkurve (154) vorgegeben sind, in die ein mit dem Betätigungselement (120) verbundener Nocken hinein ragt.

19. Chirurgisches Instrument nach einem der Ansprüche 14 bis 18, **dadurch gekennzeichnet, dass** ein Auswurfendanschlag (160) vorgesehen ist, mittels dem die Bewegung der Auswurfeinrichtung (46) in distaler Richtung begrenzbar ist und die Auswurfeinrichtung (46) mittels einer Rückstellfedereinrichtung (132) von einer Auswurfendanschlagstellung in die Ausgangsstellung verlagerbar ist, wobei gleichzeitig die Auswurfeinrichtung (46) hinter einen im Aufnahmeabschnitt (106) aufgenommenen Anker (6) positionierbar ist.

## Claims

1. Anchor arrangement (2) for surgical tissue repair, such as in particular for repair of a meniscus tear (R),
comprising at least one first anchor (4) and a second anchor (6), which are movable along a hollow needle (14) for placement in each case on a tissue (G) which is to be repaired, and connected to each other via a seam element (8),
wherein the first anchor (4) and the second anchor (6) each extend between a distal end (22) and a proximal end (24), and form a guide surface (26) on the outside thereof, for forming an at least partial contact with an inner side (28) of a hollow needle (14),
and deflection means (38) are provided on the anchors (4, 6), by means of which a torque (MD) can be applied at least in part to the anchors (4, 6) during or after setting,
**characterised in that** at least one of the anchors (4, 6) comprises at least two anchor sections (50, 52) which are movable relative to each other and which can be pivoted relative to each other between a folded position, in which the guide surface (26) spans a cross-section (Q), deviating from a circular profile, and an unfolded position, and the at least two anchor sections (50, 52) are connected to one another by means of a joint (54).

2. Anchor arrangement according to claim 1, **characterised in that** the guide surface (26), in the folded position of the anchor sections (50, 52) movable relative to one another, forms a profile which is heart-shaped, oval, or egg-shaped, in the sense of a circumferential convex profile.

3. Anchor arrangement according to claim 1 or 2, **characterised in that** the at least two anchor sections (50, 52) can be pre-tensioned in the direction of the unfolded position, wherein the seam element (8) is deflected at the anchor sections (50, 52) in such a way that they can be subjected to an unfolding torque.

4. Anchor arrangement according to any one of claims 1 to 3, **characterised in that** the at least two anchor sections (50, 52) are supported in the unfolded position in each case by an end stop of the respective other anchor section (52, 50).

5. Anchor arrangement according to any one of claims 1 to 4, **characterised in that** the at least two anchor sections (50, 52) form, in the folded position, at the proximal end (24) of at least one of the anchors (4, 6), a common spread receiver (70), which can be spread by imposition of force by a spreading means (72).

6. Surgical instrument (16) with an anchor arrangement (2) according to any one of claims 1 to 5, for surgical tissue repair, such as in particular for repair of a meniscus tear (R),
with a hollow needle (14) which forms at a distal end a needle tip (18),
and an ejection device (46), with an ejection element (44) which can be moved inside the hollow needle (14), by means of which the first and second anchors (4, 6) can be moved along the hollow needle (14),
**characterised in that** the hollow needle (14) comprises at the needle tip (18) a closed circumferential or closable cross-section (Q), and exhibits, at least in the direction towards the needle tip (18), a cross-section (Q) deviating from a circular profile.

7. Surgical instrument according to claim 6, **characterised in that** the hollow needle (14) comprises a longitudinal slot (100), and two opposing edges (102) of the longitudinal slot (100), in the state not subjected to load, are in contact with one another at the needle tip (18).

8. Surgical instrument according to claim 7, **characterised in that** the edges (102) can be widened apart by pressing in of a section of the seam element (8) or of the anchors (4, 6).

9. Surgical instrument according to any one of claims 6 to 8, **characterised in that,** by way of the by the first and the second anchor (4, 6), the needle tip (18) can in each case be closed flush with the anchor arrangement (2) accommodated therein.

10. Surgical instrument according to any one of claims 6 to 9, **characterised in that** tensioning means are provided at the hollow needle (14) for tensioning the seam element (8), which comprise a slide element (104) which can be moved along the hollow needle (14), at which the seam elements (8) is deflected and which is pre-tensioned in the proximal direction.

11. Surgical instrument according to any one of claims 6 to 10, **characterised in that** the hollow needle (14) comprises at the needle tip (18) a distal section (108) and a receiver section (106) arranged proximally thereto, which exhibits a widened cross-section in relation to the distal section (108).

12. Surgical instrument according to claim 11, **characterised in that,** in the widened cross-section, the ejection element (44) can be guided past the second anchor (6).

13. Surgical instrument according to claim 11 or 12, **characterised in that** securing means are provided in the proximal receiver section (106), by means of which the second anchor (6) can be supported in both axial directions.

14. Surgical instrument according to any one of claims 6 to 13, **characterised in that** a gear wheel drive element (124) is provided for controlling the movement sequences of the ejection device (46), and the gear wheel drive element (124) exhibits a predetermined transmission ratio between a movable actuation element (120) and the ejection element (44).

15. Surgical instrument according to claim 14, **characterised in that** the anchors (4, 6) can be supported in a setting readiness position in the proximal direction by the ejection device (46), and that its movement in the proximal direction is thereby blocked.

16. Surgical instrument according to claim 14 or 15, **characterised in that** the actuation element (120) can be brought in contact with an end stop (152), and in this situation the ejection device (46) is arranged in an end stop position, which corresponds to the setting readiness position of the anchor (4, 6) which in each case is guided together with it.

17. Surgical instrument according to claim 16, **characterised in that** the actuation element (120) can be rotated, with the separation of the gear wheel drive element (124) from the end stop position, into a release position, in which the actuation element (120) can be moved further in the setting position (RS).

18. Surgical instrument according to claims 16 or 17, **characterised in that** the end stop position and the release position are predetermined by a control curve (154), into which a cam projects, connected to the actuation element (120).

19. Surgical instrument according to any one of claims 14 to 18, **characterised in that** an ejection end stop (160) is provided, by means of which the movement of the ejection device (46) can be limited in the distal direction, and the ejection device (46) can be moved by means of a resetting spring device (132) from an ejection end stop position into the initial position, wherein, at the same time the ejection device (46) can be positioned behind an anchor (6) accommodated in the receiver section (106).

## Revendications

1. Dispositif d'ancrage (2) destiné à la réparation chirurgicale de tissus, comme notamment la réparation d'une déchirure méniscale (R),
comprenant au moins un premier ancrage (4) et un second ancrage (6) qui peuvent coulisser le long d'une aiguille creuse (14) pour la mise en place respective au niveau d'un tissu à réparer (G) et qui sont reliés entre eux par un élément de suture (8),
dans lequel le premier ancrage (4) et le second ancrage (6) s'étendent chacun entre une extrémité distale (22) et une extrémité proximale (24) et forment sur leur côté extérieur une surface de guidage (26) destiné à l'appui au moins partiel sur un côté intérieur (28) d'une aiguille creuse (14),
et, au niveau des ancrages (4, 6), des moyens de déviation (38) sont prévus, au moyen desquels les ancrages (4, 6) peuvent être soumis au moins en partie à un couple (MD) pendant ou après la mise en place,
**caractérisé en ce que** au moins un des ancrages (4, 6) comprend au moins deux parties d'ancrage (50, 52), mobiles relativement l'une par rapport à l'autre, qui peuvent pivoter relativement l'une par rapport à l'autre entre une position repliée, dans laquelle la surface de guidage (26) présente une section transversale (Q) différente d'un profil circulaire, et une position dépliée, et les au moins deux parties d'ancrage (50, 52) sont reliées l'une avec l'autre par une articulation (54).

2. Dispositif d'ancrage selon la revendication 1, **caractérisé en ce que** la surface de guidage (26) réalise, dans la position repliée des parties d'ancrage (50, 52) mobiles entre elles, un profil en forme de coeur, oval ou en forme d'oeuf dans le sens d'un profil convexe circonférentiellement.

3. Dispositif d'ancrage selon la revendication 1 ou 2, **caractérisé en ce que** les au moins deux parties d'ancrage (50, 52) peuvent être précontraintes en direction de la position dépliée, l'élément de suture (8) étant dévié sur les parties d'ancrage (50, 52) de sorte que celles-ci peuvent être soumises à un couple de dépliage.

4. Dispositif d'ancrage selon l'une des revendications 1 à 3, **caractérisé en ce que** les au moins deux parties d'ancrage (50, 52) sont supportées chacune par une butée d'extrémité de l'autre partie d'ancrage (52, 50) dans la position dépliée.

5. Dispositif d'ancrage selon l'une des revendications 1 à 4, **caractérisé en ce que** les au moins deux parties d'ancrage (50, 52), dans la position repliée, forment un logement d'écartement (70) commun au niveau de l'extrémité proximale (24) d'au moins un des ancrages (4, 6), logement qui peut être écarté par l'application d'un moyen d'écartement (72).

6. Instrument chirurgical (16) comprenant un dispositif d'ancrage (2) selon l'une des revendications 1 à 5, destiné à la réparation chirurgicale de tissus, comme notamment la réparation d'une déchirure méniscale (R), comprenant une aiguille creuse (14) qui, au niveau d'une extrémité distale, réalise une pointe d'aiguille (18),
et un dispositif d'éjection (46) muni d'un élément d'éjection (44) mobile à l'intérieur de l'aiguille creuse (14), élément au moyen duquel les premier et deuxième ancrages (4, 6) peuvent coulisser le long de l'aiguille creuse (14),
**caractérisé en ce que** l'aiguille creuse (14) comprend une section transversale (Q) pouvant être fermée ou circonférentiellement fermée au niveau de la pointe d'aiguille (18), et comprend une section transversale (Q) différente d'un profil circulaire au moins vers la pointe d'aiguille (18).

7. Instrument chirurgical selon la revendication 6, **caractérisé en ce que** l'aiguille creuse (14) présente une rainure longitudinale (100) et deux bords opposés (102) de la rainure longitudinale (100) sont situés l'un contre l'autre au niveau de la pointe d'aiguille (18) dans l'état non contraint.

8. Instrument chirurgical selon la revendication 7, **caractérisé en ce que** les bords (102) peuvent s'écarter en pressant une partie de l'élément de suture (8) ou l'ancrage (4, 6).

9. Instrument chirurgical selon l'une des revendications 6 à 8, **caractérisé en ce que** la pointe d'aiguille (18) peut être fermée de manière affleurante par les premier et deuxième ancrages (4, 6) du dispositif d'ancrage (2) reçu dans celle-ci.

10. Instrument chirurgical selon l'une des revendications 6 à 9, **caractérisé en ce que,** au niveau de l'aiguille creuse (14), des moyens de contrainte sont prévus pour contraindre l'élément de suture (8) et comprennent un élément de coulissement (104) mobile le long de l'aiguille creuse (14), élément de coulissement sur lequel est dévié l'élément de suture (8) et est précontraint dans la direction proximale.

11. Instrument chirurgical selon l'une des revendications 6 à 10, **caractérisé en ce que,** au niveau de la pointe d'aiguille (18), l'aiguille creuse (14) comprend une partie distale (108) et une partie de réception (106) disposée de manière proximale à la partie distale, la partie de réception comprenant une section transversale élargie par rapport à la partie distale (108).

12. Instrument chirurgical selon la revendication 11, **caractérisé en ce que,** dans la section transversale élargie, l'élément d'éjection (44) peut être guidé au-delà du deuxième ancrage (6).

13. Instrument chirurgical selon la revendication 11 ou 12, **caractérisé en ce que,** dans la partie de réception (106) proximale, des moyens de fixation sont prévus, au moyen desquels le deuxième ancrage (6) peut être supporté dans les deux directions axiales.

14. Instrument chirurgical selon l'une des revendications 6 à 13, **caractérisé en ce qu'**il est prévu une transmission par engrenage (124) pour commander les séquences de mouvement du dispositif d'éjection (46) et la transmission par engrenage (124) présente un rapport de transmission prédéterminé entre un élément d'actionnement mobile (120) et l'élément d'éjection (44).

15. Instrument chirurgical selon la revendication 14, **caractérisé en ce que** les ancrages (4, 6) peuvent être supportés dans une position de mise en place dans la direction proximale par le dispositif d'éjection (46) et dont le mouvement dans la direction proximale est ainsi verrouillée.

16. Instrument chirurgical selon la revendication 14 ou 15, **caractérisé en ce que** l'élément d'actionnement (120) est appliqué sur une butée d'extrémité (152) et le dispositif d'éjection (46) est disposé ainsi en position de butée d'extrémité, qui correspond à la position de mise en place d'un ancrage (4, 6) associé respectif.

17. Instrument chirurgical selon la revendication 16, **caractérisé en ce que** l'élément d'actionnement (120) peut être rotatif dans une position libre par la séparation de la transmission à engrenage (124) depuis la position de butée d'extrémité, position libre dans laquelle l'élément d'actionnement (120) peut être déplacé encore dans une direction de mise en place (RS).

18. Instrument chirurgical selon la revendication 16 ou 17, **caractérisé en ce que** la position de butée d'extrémité et la position libre sont prévues par une came de commande (154), dans laquelle une came reliée avec l'élément d'actionnement (120) s'étend vers l'intérieur.

19. Instrument chirurgical selon l'une des revendications 14 à 18, **caractérisé en ce qu'**il est prévu une butée d'éjection (160) limitant le mouvement du dispositif d'éjection (46) dans la direction distale, et le dispositif d'éjection (46) peut être déplacé au moyen d'un dispositif à ressort de rappel (132) depuis une position de butée d'éjection dans une position de départ, le dispositif d'éjection (46) pouvant simultanément être positionné derrière un ancrage (6) reçu dans la partie de réception (106).
